(19)

Europäisches
Patentamt

European
Patent Office

Office européen
des brevets

(11) **EP 4 553 173 A1**

(12) **EUROPEAN PATENT APPLICATION**

(43) Date of publication:
14.05.2025 Bulletin 2025/20

(21) Application number: 23208165.3

(22) Date of filing: 07.11.2023

(51) International Patent Classification (IPC):
*C12Q 1/6883* (2018.01)     *G01N 33/50* (2006.01)

(52) Cooperative Patent Classification (CPC):
**C12Q 1/6883; G01N 33/50;** C12Q 2600/158

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Universitätsmedizin der Johannes Gutenberg-Universität Mainz 55131 Mainz (DE)**

(72) Inventors:
 • **SCHUPPAN, Detlef**
   **65197 Wiesbaden (DE)**
 • **SURABATTULA, Rambabu**
   **55128 Mainz (DE)**
 • **SCHATTENBERG, Jörn**
   **55268 Nieder-Olm (DE)**

(74) Representative: **Patentanwälte Dr. Keller, Schwertfeger**
   **Partnerschaft mbB**
   **Westring 17**
   **76829 Landau (DE)**

(54) **MARKERS FOR DETECTING AND MONITORING LIVER FIBROSIS**

(57)     The present invention relates to methods for detecting the presence and/or predicting the severity or stage of chronic liver fibrosis in an individual, comprising the analysis of the expression levels of at least two markers selected from the group consisting of matricellular fibrosis marker thrombospondin-2 (TSP2), insulin like growth factor binding protein 7 (IGFBP7), growth differentiation factor 15 (GDF-15), macrophage marker CD163, matricellular fibrosis markers thrombospondin-4 (TSP4) or ADAMTS9 in a biological sample obtained from said individual. The invention also relates to methods for predicting the efficacy of a treatment of a chronic liver fibrosis in an individual, comprising the analysis of said at least two markers. The invention furthermore relates to diagnostic or therapeutic test systems, comprising reagents and material that allow the analysis of said at least two markers. The diagnostic or therapeutic test system according to the present invention are suitable for use in the diagnosis of chronic liver fibrosis in an individual. The invention further relates to a composition of said at least two markers for use in the treatment of chronic liver diseases by predicting the response to treatment with a candidate agent and/or monitoring the fibrogenesis of chronic liver fibrosis under such therapy, or with, e.g., nutritional or life style intervention. The invention also relates to methods of selecting or qualifying a pharmaceutically active candidate agent or a nutritional or life style intervention for inhibiting the progression or inducing the regression of a chronic fibrotic liver disease in a patient, and a candidate agent or intervention derived from said methods.

EP 4 553 173 A1

**Description**

The present invention relates to methods for detecting the presence and/or predicting the severity or stage of chronic liver fibrosis in an individual, comprising the analysis of the expression levels of at least two markers selected from the group consisting of matricellular fibrosis marker thrombospondin-2 (TSP2), insulin like growth factor binding protein 7 (IGFBP7), growth differentiation factor 15 (GDF-15), macrophage marker CD163, matricellular fibrosis markers thrombospondin-4 (TSP4) or ADAMTS9 in a biological sample obtained from said individual. The invention also relates to methods for predicting the efficacy of a treatment of a chronic liver fibrosis in an individual, comprising the analysis of said at least two markers. The invention furthermore relates to diagnostic or therapeutic test systems, comprising reagents and material that allow the analysis of said at least two markers. The diagnostic or therapeutic test system according to the present invention are suitable for use in the diagnosis of chronic liver fibrosis in an individual. The invention further relates to a composition of said at least two markers for use in the treatment of chronic liver diseases by predicting the response to treatment with a candidate agent and/or monitoring the fibrogenesis of chronic liver fibrosis under such therapy, or with, e.g., nutritional or life style intervention. The invention also relates to methods of selecting or qualifying a pharmaceutically active candidate agent or a nutritional or life style intervention for inhibiting the progression or inducing the regression of a chronic fibrotic liver disease in a patient, and a candidate agent or intervention derived from said methods.

The marker combinations according to the present invention are suitable for detecting and monitoring different liver disease etiologies, such as non-alcoholic steatohepatitis (NAFLD/NASH), alcoholic liver disease (ALD), viral hepatitis B, C or D, autoimmune or genetic liver diseases including primary biliary cholangitis (PBC) or primary sclerosing cholangitis (PSC), common variable immunodeficiency (CID) liver disease, or infectious liver disease like schistosomiasis. Apart from predicting the stage or severity of liver fibrosis, these test combinations also reflect the activity and progression of the fibrotic process and serve as companion diagnostics for antifibrotic drug development or personalized antifibrotic therapies.

Organ fibrosis, i.e., scarring, is responsible for at least 50% of the morbidity and mortality of chronic diseases worldwide. Fibrosis is characterized by excess deposition of scar tissue (extracellular matrix, ECM) at the expense of functional tissue. Fibrosis usually results from chronic inflammation, in the body's attempt to prevent organ disintegration of 'wounds that do not heal'. Causes are multiple, including chronic infections, autoimmune or metabolic diseases.

Ongoing fibrosis leads to progressive scarring and functional failure of the affected organ. Moreover, advanced fibrosis is associated with grave vascular changes in and outside of the affected organs. Finally, scarring can highly increase the risk of cancer in the affected organ. Prominent examples of advanced stage fibrotic diseases are liver cirrhosis, lung or kidney fibrosis, systemic sclerosis, cardiac fibrosis or arteriosclerosis with multiorgan involvement. To date, there exist no approved antifibrotic therapies and no highly predictive, sensitive and approved non-invasive fibrosis biomarkers. This invention describes combinations of serum (plasma) biomarkers that are uniquely suited to predict fibrosis stage and activity of fibrosis progression or regression, especially for fibrotic liver diseases, but with potential also for fibrotic diseases of other organs.

Tissue Fibrosis, as, e.g., seen on representative tissue sections of an affected organ, results from a dynamic and ongoing process of fibrogenesis (fibrosis progression) or fibrolysis (fibrosis regression), even in advanced fibrosis stages. The balance between de novo scar tissue formation and deposition (fibrogenesis) and its removal (fibrolysis) over a defined period of time finally determines the extent of fibrosis or its regression. In the liver, scar tissue is mainly produced by activated hepatic stellate cells and activated (myo-) fibroblasts, but also other cell types such as activated endothelial cell and activated biliary epithelia (cholangiocytes) contribute. Various other cell types, 'upstream' of these cells, such as monocytes-macrophages, lymphocytes or activated (biliary) progenitors (cholangiocytes), and multiple mediators such as cytokines and chemokines produced in the inflammatory or fibrotic microenvironment, drive or contribute to the fibrotic process. Notably, the mechanisms underlying fibrosis in other organs than liver, such as kidney and lungs, blood vessels or skin, share a high degree of similarity, although also organ-specific mechanisms come in into play. Therefore, antifibrotic drug development for a certain organ, a field of expanding relevance for patients, for biotech and pharma may also be useful for antifibrotic therapy of other organs. The sensitive and specific, early detection of fibrotic diseases in clinically asymptomatic subjects (patients) on a broad scale and based on a cheap, non-invasive serum (plasma) test could allow to institute measures early enough to prevent progression to organ failure such as cirrhosis of the liver, pulmonary or renal failure. However, to date none of the currently described 'fibrosis markers' fulfils these criteria.

Apart from early detection and prevention of progressive fibrosis, a sensitive and specific serum (plasma) test of fibrosis could dramatically speed up the clinical development of specific and highly active antifibrotic drugs. At present, this is only possible in large and well stratified patient cohorts treated with a single agent over one year or longer and monitored by quantification of scar tissue in organ biopsies at baseline and at the end of treatment, usually accompanied by parameters reflecting organ function as a surrogate - such organ function measures are reasonable readouts for lungs and kidneys, but unreliable for liver. In addition, such test would reflect not only the stage of fibrosis, where much scar tissue deposited in the organ is usually associated by higher turnover of scar tissue which then may increase biomarker levels, but rather the dynamic process of fibrogenesis, i.e., the production of scar components in the affected organ. Serum (plasma) fibrosis marker development is most advanced for diseases of the liver, a large organ where active inflammation and fibrosis is paralleled by elevations of numerous serum (plasma) proteins/peptides, enzyme activities, or miRNAs. In fact, most recent biomarker research has been performed in fatty liver disease and (fibrotic) NASH (Karsdal MA, Kraus VB, Shevell D, Bay-Jensen AC, Schattenberg J, Surabattula R, Schuppan D. Profiling and targeting connective tissue remodeling in autoimmunity - A novel paradigm for diagnosing and treating chronic diseases. Autoimmun Rev 2021,20,102706, PMID: 33188918; Schuppan D, Myneni S, Surabattula R. Liquid biomarkers for fibrotic NASH - progress in a complex field. J Hepatol 2022,76-7, PMID: 34801249; Wattacheril JJ, Abdelmalek MF, Lim JK, Sanyal AJ. AGA Clinical Practice Update on the Role of Noninvasive Biomarkers in the Evaluation and Management of Nonalcoholic Fatty Liver Disease: Expert Review. Gastroenterology 2023,165,1080-88., PMID 37542503; Sanyal AJ, Castera L, Wong VW. Noninvasive Assessment of Liver Fibrosis in NAFLD. Clin Gastroenterol Hepatol, 2023,21,2026-39, PMID: 37062495).

WO 2017/139254 A1 describes methods of determining whether a subject has non-alcoholic fatty liver disease. The methods comprise detecting the level of at least one biomarker selected from ACY1, C7, COLEC11, CSF1R, DCN, KYNU, POR and THBS2, in a sample from the subject, wherein a level of at least one biomarker selected from ACY1, C7, COLEC11, CSF1R, DCN, KYNU, POR and THBS2 that is higher than a control level of the respective biomarker, indicates that the subject has NAFLD.

WO 2019/099706 A1 describes methods for diagnosing non-alcoholic steatohepatitis (NASH) with or without advanced liver fibrosis in a subject by detecting the levels of a variety of biomarkers diagnostic of NASH. The methods however focus on NASH and NASH-fibrosis and do go further into liver fibrosis stage and fibrosis progression in a broad spectrum of chronic liver diseases. Notably, NASH on which the two mentioned patents focus exclusively is a multifactorial disease where other serum markers can be linked to advanced liver disease or liver disease progression (related mainly to metabolism but not directly to fibrosis). Such metabolic parameters do not predict fibrosis and have no value for the other liver diseases. However, there remains a need for methods and test systems that allow the detection and monitoring of different etiologies of liver fibrosis.

WO 2022/014580 A1 describes thrombospondin-2 protein as a marker for non-alcoholic fatty liver disease, which has high sensitivity and high specificity to non-alcoholic fatty liver disease and can accurately determine a condition of non-alcoholic fatty liver disease. Only a single serum protein marker is disclosed with focus on NAFLD/NASH.

It is therefore the object of the present invention to provide improved methods, compositions and test systems that allow detecting and monitoring of liver fibrosis with high sensitivity and high specificity, in order to predict the severity or stage, as well as the activity of the fibrotic processes in a patient.

This object is solved by the methods, compositions and test systems according to the present invention, in particular by selecting a combination of at least two biological markers that consist of matricellular fibrosis marker thrombospondin-2 (TSP2), insulin like growth factor binding protein 7 (IGFBP7), growth differentiation factor 15 (GDF-15), macrophage marker CD163, matricellular fibrosis markers thrombospondin-4 (TSP4) or ADAMTS9.

The expressions "marker", "biological marker" or "biomarker" are used herein interchangeably and refer to a biological molecule that relates to a molecule or substance present in a biological sample that can be determined to make a connection with a biological condition or stage.

As shown herein, the markers and marker combinations of the present invention have in common that they are very similar in their predictive value for any kind of liver disease, and thus qualify as direct markers of fibrosis. In some aspects, the invention provides a significantly enhanced serum (plasma) fibrosis marker test, in order to detect and quantify a decrease of fibrogenesis due to early intervention and especially due to an antifibrotic pharmacological agent. The marker combinations according to the present invention allow small proof of concept studies with novel pharmacological agents or even drug combinations, and also permit a personalized therapy for each patient by regularly monitoring fibrogenesis and fibrolysis in the blood. The marker combinations of the present invention thus help to prevent disease progression to difficult-to-treat or untreatable advanced stages, support speedy development of effective antifibrotics, including repurposing of out-of-license drugs used in combinations, and provide a measure for personalized antifibrotic therapy. Since liver fibrosis is the most important determinant of long-term outcome and mortality, it is important to determine the degree of fibrosis and risk for progression in order to institute early interventions and guide treatment strategies to mitigate the development of cirrhosis and the morbidity and mortality associated with it. The marker combinations of the present invention, apart from less predictive laboratory testing and imaging studies can be used to determine a potential risk for progression of fibrotic liver diseases without recourse to liver biopsy.

In a first aspect, the present invention relates to a method for detecting the presence and/or predicting the severity or stage of chronic liver fibrosis in an individual, comprising the steps of (a) determining the expression levels of at least two markers selected from the group consisting of matricellular fibrosis marker thrombospondin-2 (TSP2), insulin like growth factor binding protein 7 (IGFBP7), growth differentiation factor 15 (GDF-15), macrophage marker CD163, matricellular fibrosis markers thrombospondin-4 (TSP4) or ADAMTS9 in a biological sample obtained from said individual, (b) comparing the expression levels of said at least two markers in said biological sample with the expression levels of said at least two markers in a sample obtained from a healthy control or a control sample, wherein an increase of the expression of said at least two markers in said individual compared to the healthy control or control sample is indicative for the presence, severity and/or stage of chronic liver fibrosis in said individual.

In a preferred embodiment, a preferred marker combination comprises the presence of at least the biomarker TSP2 in combination with one or more of the biomarkers IGFBP7 and/or CD163. In a further preferred embodiment, the invention relates to a marker combination comprising at least the three markers consisting of IGFBP7, TSP2 and CD163.

In other preferred embodiments, GDF-15 and/or TSP4 is used either alone, or in combination with IGFBP7, TSP2 and/or CD163

In any of the embodiments described herein, the biological sample may be a blood sample. Preferably, the blood sample is selected from a serum sample or a plasma sample. In alternative embodiments, the biological sample can be a biopsy material obtained from the individual or subject to be treated.

A "biological sample" as used herein refers to any material, biological fluid, tissue or cells obtained or otherwise derived from the individual. This includes blood including whole blood, leukocytes, peripheral blood mononuclear cells, buffy coat, plasma, and serum. This also includes experimentally separated fractions of all of the preceding. For example, a blood sample can be fractionated into serum, plasma, or into fractions containing particular types of blood cells, such as red blood cells or white blood cells (leukocytes). In some embodiments, a sample can be a combination of samples from an individual, such as a combination of a tissue and fluid sample. A biological sample also includes materials containing homogenized solid material, such as from a stool sample, a tissue sample, or a tissue biopsy.

A biological sample can also include materials derived from a tissue culture or a cell culture. Any suitable methods for obtaining a biological sample can be employed; exemplary methods include, e.g., phlebotomy, saliva, swab (e.g., buccal swab), peritoneal fluid, urine, and a fine needle aspirate biopsy procedure. Samples can also be collected, e.g., by micro dissection (e.g., laser capture micro dissection (LCM) or laser micro dissection (LMD)), bladder wash, smear (e.g., a PAP smear), or ductal lavage. A biological sample obtained or derived from an individual includes any such sample that has been processed in any suitable manner after being obtained from the individual.

As used herein, "individual" or "subject" are used interchangeably to refer to a test subject or patient. The individual can be a mammal or a non-mammal. In various embodiments, the mammal is a human or animal. A healthy or normal individual is an individual in which the disease or condition of interest (one or more of the liver disease etiologies described herein) is not detectable by diagnostic methods.

The terms "diagnosing" or "diagnosis" encompass, with respect to a particular disease or condition, the initial detection of the disease; the characterization or classification of the disease; the detection of the progression, remission, or recurrence of the disease; and the detection of the disease response after the administration of a treatment or therapy to the individual.

The "expression level" of a marker as described herein relates to the level of a biomarker present in a sample to be investigated. The expression level can be a quantity, volume or concentration. The expression level can be measured by any suitable method available that allows the determination of the quality or quantity of the concentration of the respective marker in the sample. In preferred embodiments, the material that is used to determine the expression level can be a nucleic acid such as DNA, RNA, or a protein. The expression level can therefore be measured at different levels of protein expression, including, but not limited to gene expression, transcription, translation or protein expression. The marker molecule of the present invention may be present as wildtype, in modified or un-modified form in the biological sample, preferably serum or plasma sample. The invention also covers human or non-human variants of any of the markers described in the present invention. The invention furthermore comprises modified versions of the markers that maintain their biological activity or significance.

In some embodiments, measurements are made using any analytical method for detecting the biomarkers in a biological sample, e.g., a method or test that indicates the presence, absence, absolute amount or concentration, relative amount or concentration, titer, level or ratio of measured levels or the like of, for, or corresponding to the marker in the biological sample. The particular nature of the expression level of each marker depends on the specific design and components of the particular analytical method employed to detect the respective marker.

A "healthy control", "healthy control subject" or control sample refers to the level of the marker molecule in a sample from an individual that does not have the disease or condition, or from an individual that is not suspected of having the disease or condition. It is not required that the control expression level of a target marker molecule is to be determined each time the methods of the invention are carried out, and maybe a previously determined expression level that is used as a reference of result to determine whether the level in a particular sample is higher or lower than a normal level.

As shown herein, the methods, compositions and test systems of the present invention allow monitoring and diagnosis of a variety of fibrotic liver diseases, in particular chronic liver fibrosis in a mammal, preferably a human individual. In one aspect, the chronic liver fibrosis relates to a liver disease selected from the group consisting of as non-alcoholic steatohepatitis (NAFLD/NASH), alcoholic liver disease (ALD), alcoholic hepatitis (AH), viral hepatitis B, C or D, post-transplant liver disease, common variable immunodeficiency (CVID), autoimmune hepatitis (AIH), genetic liver disease, primary biliary cholangitis (PBC), primary sclerosing cholangitis (PSC), or infectious liver disease like schistosomiasis. Each of the mentioned liver disease etiologies is characterized by an ongoing fibrotic progress or fibrogenesis of the pathological liver. For each of the respective liver disease, a marker combination of at least two biomarkers is preferred such that the diagnostic methods of the invention allow to determine the stage or severity of liver fibrosis at high specificity and high sensitivity within a given time course.

In preferred embodiments, a prognostic score for each of the liver disease is determined by incorporating clinical biological parameters and values of the determined expression levels of at least two markers. This is achieved by applying a comprehensive multivariable analysis employing logistic regression. Preferably, the logistic regression is constructed using a backward stepwise approach by iteratively eliminating variables that lack statistical significance or do not substantially enhance the model's predictive capability.

In preferred embodiments, predictive values of the fibrosis markers described herein and various marker combinations are used to predict the presence of a fibrotic liver disease in an individual. In those embodiments, the calculation results in an area under receiver operating characteristics (AUROC), wherein a value > 0.8 defines a marker having high specificity and high sensitivity. This means that a AUROC of > 0.8, preferably > 0.9 qualifies the marker or marker combination as biological marker that is suitable for monitoring the progress of liver fibrosis in an individual, and thus allows the diagnosis and prediction of the severity and/or stage of fibrogenesis, as well as fibrosis progression or regression in the individual.

The present invention makes use of algorithms that include clinical biological parameters (in short: clin-biol parameters) that contribute to the high AUROC values obtained with the markers and marker combinations according to the present invention. In preferred embodiments, the clinical biological parameters include platelets and AST (aspartate amino transferase). In alternative embodiments, the clinical biological parameters include, but are not limited to age, albumin, platelets, AST (aspartate amino transferase), and GGT (gamma-glutamyl transpeptidase).

In preferred embodiments two biomarkers are combined with the clinical biological parameters in order to obtain the predictive values of said marker combination. Preferably, cross validations are carried out and average to generate a plot that reflects sensitivity (true positive rate) and specificity (false positive rate) using the average accuracy based on > 50, preferably around 100 cross validations, to differentiate histologically (matched biopsy-derived) no or mild fibrosis (stage F0-F1) from significant (stage F2-F4) fibrosis, or no/mild/moderate fibrosis (stage F0-F2) from severe fibrosis/cirrhosis (stage F3-F4), or cirrhosis (stage F4 from stage F0-F3). In NASH, an additional differentiation is used, i.e., NASH at risk (fibrosis stage F2-F4 plus significant liver inflammation and hepatocyte damage as determined by a histological NAS score $\geq$4) vs non-risk NAFLD/NASH (fibrosis stage F0-F1, NAS score 0-3). The algorithm that is used in the calculation for a particular marker depends inter alia on the applied test system and pathological values. It also depends to a lesser degree on the etiology of the liver disease. One example of a general algorithm used in the present invention can be described by the following formula, wherein f designates the factor: f1*TSP2 (ng/ml) + f2* IGFBP7 (ng/ml) + f3* CD163 (ng/ml). In preferred embodiments the algorithm is supplemented by f4*AST (U/L) + f5*PLT ($10^9$/mL) to reflect the clinical biological parameters of aspartate aminotransferase (AST) and platelets (PLT). The complete formula then can be summarized as follows:

$$f1{*}TSP2\ (ng/ml) + f2{*}\ IGFBP7\ (ng/ml) + f3{*}\ CD163\ (ng/ml) + f4{*}AST\ (U/L) + f5{*}PLT\ (10^9/mL).$$

In preferred embodiments, the multivariate model is a logistic regression machine-learning algorithm that can be automatable. In some embodiments, the prognostic score is validated according to random forest machine-learning algorithm. In some embodiments, the non-invasive prognostic method is computer-implemented.

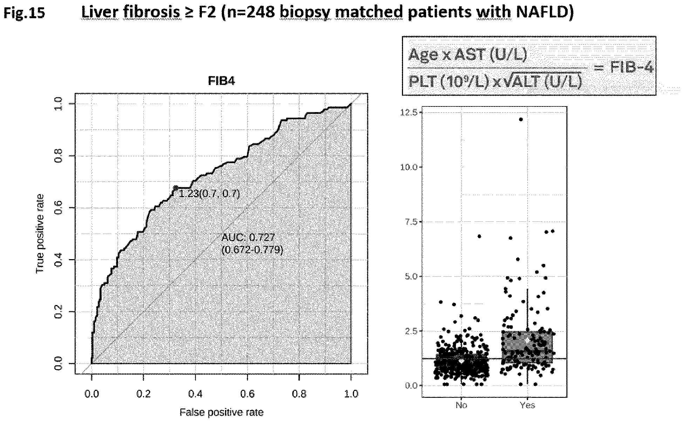

In some embodiments, the FIB-4 score is used, based on age and clinical laboratory parameters in patients suffering from a liver fibrosis disease described herein. The FIB-4 is a recognized parameter to determine the risk for advanced fibrosis. The FIB-4 score can be calculated using the formula as shown in Figure 15.

For example, individuals who have simple steatosis or a FIB-4 < 1.3 and LSM < 8 Kpa are at low risk for advanced fibrosis or decompensation and can be managed conservatively with lifestyle modification. Individuals who have a FIB-4 between 1.30 - 2.67 and LSM between 8-15 Kpa, have an intermediate risk of advanced fibrosis and should be followed closely and evaluated on regular intervals with repeated Fibroscan® (liver stiffness measurement, LSM), platelet counts and liver panel, or advanced imaging modalities such as magnetic resonance elastography) MRE to further define their disease progression and institute appropriate measures. Individuals with a FIB-4 > 2.67 and LSM > 15 Kpa, or LSM > 20 Kpa are at high risk of cirrhosis and decompensation. They should undergo aggressive therapy. However, these markers and marker combinations have a low predictive value for the different stages and severities of liver fibrosis (except for a good prediction of established cirrhosis by Fibroscan/LSM or MRE).

Since the AUROC value of a combination of at least two markers described herein is > 0.8, the inventive marker combination qualifies as an excellent screening biomarker. For example, the combination of TSP2 and IGFB7 has a unique predictive value (AUROC 0.91) to diagnose advanced fibrosis. The combination of TSP2, IGFB7 and CD163 has the best predictive value (AUROC 0.838) to diagnose significant fibrosis (F2-F4) and advanced fibrosis (F3) or cirrhosis (F4).

The parameters and algorithms used in context of the markers of the present invention have at least as great, if not greater, value as dynamic biomarkers of fibrosis progression or fibrosis regression (serum half life approximately 1-2 days) and are therefore ideal companion biomarkers for clinical studies and personalized antifibrotic therapy.

In alternative embodiments, the combination of TSP2, IGFB7 and CD163 (and TSP4) has provided the best predictive value (AUROC 0.791) to diagnose significant fibrosis such as in patients with primary sclerosing cholangitis (PSC), a liver disease with very heterogeneous patterns of fibrosis, resulting in a huge sampling error of liver biopsy. The same marker combination is also highly efficient in individuals suffering from alcohol-induced cirrhosis. TSP4 is a matricellular protein related to TSP2, being related to epithelial-mesenchymal transition (EMT), a driver of biliary fibrosis, contributes to prediction in, e.g., biliary fibrotic liver diseases.

For monitoring the fibrotic progress or stage of non-alcoholic steatohepatitis (NAFLD/NASH), the marker combination of TSP2, IGFB7 and CD163 is preferred. For monitoring the fibrotic progress or stage of autoimmune hepatitis (AIH), the marker combination of TSP2, IGFB7 and CD163 is preferred. For common variable immunodeficiency (CVID), the marker combination of TSP2, IGFB7 and CD163 is preferred. For monitoring the fibrotic progress or stage of primary biliary cholangitis (PBC) and primary sclerosing cholangitis (PSC), the marker combination of TSP2, IGFB7 and CD163 is preferred. For monitoring the fibrotic progress or stage of the treatment of PBC and PSC, the marker combination of TSP2, IGFB7, CD163 or TSP4 is preferred. Preferred marker combinations and their connection to the chronic fibrotic liver diseases as validated in the present invention can be summarized as follows:

| Disease | TSP2 | IGFBP7 | GDF-15 | CD163 | TSP4 |
|---|---|---|---|---|---|
| NAFLD/NASH | X | X | | X | |
| ALD/AH | X | X | | X | |
| Hepatitis C | X | X | X | X | |
| Post transplant | X | X | X | X | |
| AIH | X | X | | X | |
| CVID | X | X | | X | |
| PBC / PSC | X | X | | X | X |

For example, two markers selected from TSP2, IGFB7, CD163, TSP4 can be used, in order to analyse fibrotic progression of PBC or PSC. Since the combination of at least two markers selected from TSP2, IGFB7 and CD163 provides high predictive scores for all of the fibrotic diseases described herein, the combination of TSP2, IGFB7 and CD163 is suitable in the monitoring and predicting fibrogenesis in a subject. In addition to the at least two markers of the marker combinations, the marker ADAMTS9 can be determined, in particular in type 2 diabetes individuals undergoing bariatric surgery or being overweight or morbidly obese.

The following tables summarize the ROC-analyses for different fibrotic liver diseases. The markers tested exhibit a high sensitivity and specificity in population screening for the chronic liver diseases described in the present invention.

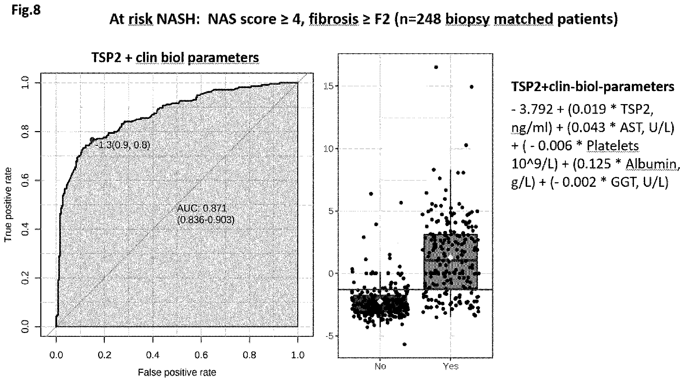

Table 1 shows a comparison of the predictive values of the single fibrosis markers and the various marker combinations to predict NASH at risk in 248 patients with non-alcoholic fatty liver disease (NAFLD) whose matched liver biopsies were staged and graded according to international criteria. Patients with non-alcoholic steatohepatitis (NASH; i.e., a NAS score $\geq$ 4) and significant liver fibrosis (stage $\geq$ 2) are at a high risk to progress to cirrhosis (and therefore classified as patients with NASH at risk). An AUROC (area under receiver operating characteristics) > 0.8 defines a good, of > 0.9 a very good screening biomarker. The superiority of the novel defined marker algorithms is evident, as also detailed in Figures 8 - 13. Clinical biological (clin-biol) parameters, i.e., selected from age, albumin, platelets, AST (aspartate aminotransferase), GGT (gamma-glutamyl transpeptidase, as detailed in Figures 8-13.

**Table 1 Summary of ROC analyses: at risk NASH**

**Features are ordered by the highest AUROC values**

| Test Result Variable(s) | AUROC | Std. Error | 95% Confidence Interval | | Asymptotic significance |
|---|---|---|---|---|---|
| | | | Lower Bound | Upper Bound | |
| TSP2+IGFBP7+CD163+clin-biol parameters | 0.884 | 0.016 | 0.832 | 0.921 | <0.0001 |
| TSP2+IGFBP7+clin-biol parameters | 0.873 | 0.017 | 0.827 | 0.914 | <0.0001 |
| TSP2+clin-biol parameters | 0.872 | 0.017 | 0.825 | 0.915 | <0.0001 |
| IGFBP7+clin-biol parameters | 0.831 | 0.020 | 0.771 | 0.881 | <0.0001 |

(continued)

**Features are ordered by the highest AUROC values**

| Test Result Variable(s) | AUROC | Std. Error | 95% Confidence Interval | | Asymptotic significance |
| --- | --- | --- | --- | --- | --- |
| | | | Lower Bound | Upper Bound | |
| TSP2 (ng/ml) only | 0.823 | 0.020 | 0.776 | 0.875 | <0.0001 |
| CD163 (ng/ml) | 0.757 | 0.023 | 0.705 | 0.814 | <0.0001 |
| IGFBP7 (ng/ml) | 0.733 | 0.024 | 0.672 | 0.801 | <0.0001 |
| FIB-4 | 0.726 | 0.024 | 0.658 | 0.778 | <0.0001 |

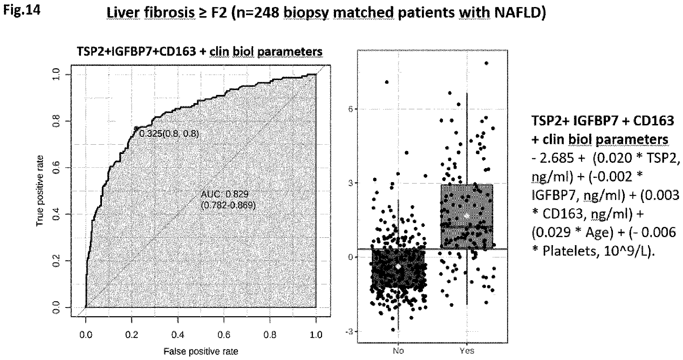

Table 2 shows a comparison of the predictive values of the single fibrosis markers and the various marker combinations to predict liver fibrosis $\geq$ stage 2 (according to Metavir: stage 0: no fibrosis, stage 4: cirrhosis) in 248 patients with non-alcoholic fatty liver disease (NAFLD) whose matched liver biopsies were staged and graded by an expert liver pathologist according to international criteria. Patients with significant liver fibrosis (stage $\geq$ 2) are at an increased risk to progress to cirrhosis, irrespective of liver disease cause. An AUROC > 0.8 defines a good, of > 0.9 a very good screening biomarker. The superiority of the novel defined marker algorithms is evident, as also detailed in Figures 14 - 16. Clinical biological (clin-biol) parameters, i.e., selected from age, albumin, platelets, AST (aspartate aminotransferase), GGT (gamma-glutamyl transpeptidase, as detailed in Figures 14-16.

**Table 2 Summary of ROC analyses: liver fibrosis $\geq$ stage 2 (patients with NAFLD)**

| Features are ordered by the highest AUROC values | | | | | |
| --- | --- | --- | --- | --- | --- |
| Test Result Variable(s) | AUROC | Std. Error | 95% Confidence Interval | | Asymptotic significance |
| | | | Lower Bound | Upper Bound | |
| **TSP2+IGFBP7+CD163+clin-biol parameters** | **0.829** | 0.022 | 0.772 | 0.885 | <0.0001 |
| **TSP2+IGFBP7+clin-biol parameters** | **0.827** | 0.022 | 0.765 | 0.881 | <0.0001 |
| **TSP2+clin-biol parameters** | **0.822** | 0.023 | 0.758 | 0.877 | <0.0001 |
| **TSP2 (ng/ml) only** | **0.817** | 0.022 | 0.759 | 0.870 | <0.0001 |
| CD163 (ng/ml) | 0.761 | 0.025 | 0.705 | 0.815 | <0.0001 |
| FIB-4 | 0.727 | 0.026 | 0.656 | 0.796 | <0.0001 |
| IGFBP7+clin-bio variables | 0.706 | 0.077 | 0.637 | 0.782 | <0.0001 |
| IGFBP7 (ng/ml) only | 0.678 | 0.028 | 0.605 | 0.744 | <0.0001 |

Tables 3 to 7 summarize the predictive algorithms for different fibrotic liver diseases using specific calculations for each marker combination. Clinical biological parameters are, for example, age, albumin, platelets, AST (aspartate aminotransferase), GGT (gamma-glutamyl transpeptidase).

**Table 3 Summary of predictive algorithms: Fibrosis progression post-liver transplantation or fibrosis regression pre vs post highly effective antiviral treatment for hepatitis C**

*Post transplant non-progressors vs progressors*
- **TSP2/IGFBP7**          = -1.72 + (0.0148 * TSP2, ng/ml) + (0.0006 * IGFBP7, ng/ml)
- **TSP2/IGFBP7/CD163**    = -2.34 + (0.011 * TSP2, ng/ml)+(0.00004 * IGFBP7, ng/ml)+ (0.00146 * CD163, ng/ml)
- **TSP2/CD163**           = -2.456 + (0.0118 * TSP2, ng/ml) + (0.0015 * CD163, ng/ml)

*Pre vs post effective antiviral treatment*
- **TSP2/CD163**           = 5.4 + (-0.028 * TSP2, ng/ml) + (-0.0034 * CD163, ng/ml)
- **TSP2/CD163/GDF15**     = 6.39 + (-0.038 * TSP2, ng/ml) + (-0.005 *CD163, ng/ml) + (0.0015 * GDF15)

**Table 4 Summary of predictive algorithms: At-risk NASH (NAS score $\geq$4 & fibrosis $\geq$F2) and liver fibrosis $\geq$F2**

*At-risk NASH*
- **TSP2+clin-biol parameters**

(continued)

*At-risk NASH*

= - 3.792+(0.019 * TSP2, ng/m))+(0.043 * AST, U/L)+(- 0.006 * Platelets 10^9/L)+(0.125 * Albumin, g/L) + (- 0.002 * GGT, U/L)

• **IGFBP7+clin-biol parameters**

= - 3.640+(0.003 * IGFBP7,ng/ml)+(0.055 * AST, U/L)+(- 0.008*Platelets. 10^9/L)+(0.124*Albumin, g/L)

• **TSP2+IGFBP7+din-biol parameters**

= - 3.885+(0.022 * TSP2, ng/ml)+(-0.003 * IGFBP7, ng/ml)+(0.048 * AST, uL)+( -0.006 * Platelets, 10^9/L) + (0.128 * Albumin, g/L)

• **TSP2+IGFBP7+CD163+clin-biol parameters**

= - 5.000+(0.014 * TSP2, ng/ml)+(-0.002 * IGFBP7,ng/ml)+(0.004 * CD163, ng/ml)+(0.039 * AST, U/L)+(- 0.006 * Platelets,

10^9/L)+(0.133 * Albumin, g/L)

*Liver fibrosis ≥F2*

• **TSP2+ IGFBP7 + CD163 + clin-biol parameters**

= - 2.685 + (0.020 * TSP2, ng/ml) + (-0.002 * )GF8P7, ng/ml) + (0.003 * CD163, ng/ml) + (0.029 * Age) + ( - 0.006 * Platelets,

10^9/L)

• **Fibrosis_T2_I7_clin_biol parameters**

= - 1.839 + (0.025 * TSP2, ngml) + (-0.002 * IGFBP7, ngml) + (0.037 * Age) + ( - 0.006 * Platelets 10^9/L)

**Table 5 Summary of predictive algorithms: No/mild vs moderate/advanced liver fibrosis (patients with NAFLD subjected to bariatric surgery and patients with common variable immunodeficiency (CVID))**

*NAFLD-Bariatric surgery*

• **TSP2/IGFBP7/CD163** = -4.76 + (0.023 * TSP2, ng/ml) + (0.019 * IGFBP7, ng/ml) + (0.00015 * CD163)

• **TSP2/IGFBP7/ADAMTS9** = -4.86 + (0.018 * TSP2, ng/ml) + (0.019 * IGFBP7, ng/ml) + (0.005 * ADAMTS9)

• **TSP2/IGFBP7/CD163/ADAMTS9** = -4.7 + (0.019 * TSP2, ng/ml) + (0.019 * IGFBP7, ng/ml)+ (-0.0003 * CD163) + (0.005 * ADAMTS9)

*Portal liver fibrosis - CVID*

• **TSP2 + IGFBP7** = -9.75 + (0.04 * TSP2, ng/ml) + (0.045 * IGFBP7, ng/ml)

**Table 6 Summary of predictive algorithms: No/mild vs moderate/advanced and mild/moderate vs advanced liver fibrosis (patients with autoimmune hepatitis (AIH) & primary sclerosing cholangitis (PSC))**

**No/mild vs moderate/advanced liver fibrosis**

*AIH*

• **TSP2/IGFBP7** = - 4.35 + (0.025 * TSP2, ng/ml) + (0.02 * IGFBP7, ng/ml)

• **TSP2/IGFBP7/CD163** = - 4.33 + (0.023 * TSP2, ng/ml) + (0.019 * IGFBP7, ng/ml) + (0.0003 * CD163, ng/ml)

*PSC*

• **TSP2/CD163** = -2.99 + (0.015 * TSP2, ng/ml) + (0.0016 * CD163, ng/ml)

• **TSP2/IGFBP7/CD163** = - 3.08 + (0.014*TSP2, ng/ml) + (0.0005 * IGFBP7, ng/ml) + (0.0016 * CD163, ng/ml)

**Mild/moderate vs advanced liver fibrosis**

*AIH*

• **TSP2/IGFBP7** = - 5.77 + (0.014 * TSP2, ng/ml) + (0.017 * IGFBP7, ng/ml)

• **TSP2/IGFBP7/CD163** = -6.046 + (0.01 * TSP2, ng/ml) + (0.017 * IGFBP7, ng/ml) + (0.0007 * CD163, ng/ml)

*PSC*

• **TSP2/IGFBP7/CD163** = - 3.94 + (0.018 * TSP2, ng/m))+(0.009 * IGFBP7, ng/ml)+(0.0016 * CD163, ng/ml)

• **TSP2/IGFBP7/CD163/TSP4** = - 3.94 + (0.018 * TSP2, ng/ml) + (0.0008 * IGFBP7, ng/ml) + (0.0016 * CD163, ng/ml) + (0.00004 * TSP2, ng/ml)

**Table 7 Summary of predictive algorithms: Healthy controls vs Child-Pugh A alcoholic cirrhosis**

> **_Healthy controls vs Child-Pugh A_**
> • **TSP2/IGFBP7** = -18.73+(0.056 * TSP2, ng/ml)+(0.06 * IGFBP7, ng/ml)
> • **TSP2/IGFBP7/CD163** = -1030.6+(5.01 * TSP2, ng/ml)+(4.59 * IGFBP7, ng/ml)+(-0.53 * CD163, ng/ml)

The marker levels obtained by the methods and compositions of the present invention can be determined by proprietary assays. In some aspects the assays are well validated in terms of reproducibility, linearity, recovery of purified target protein - according to standards set by the regulatory authorities. If other test systems should be established or used with no or lower validation, the algorithms may be adapted to the specific utilized test systems. Moreover, algorithms, mainly based on TSP2, IGFBP7 and CD163 do usually differ for the different liver disease etiologies as reflected by the general formula mentioned above.

In summary, the present invention provides novel and improved marker combinations consisting of at least two markers to achieve high specificity and high sensitivity using the predictive algorithms. Using the predictive algorithms underlying the present invention, high predictive scores can be obtained. It is therefore evident that the marker combinations of the present invention are suitable to be used in methods for predicting the efficiency of treatment of a chronic liver fibrosis in an individual, and therefore the use of the markers is of beneficial effect for the patient.

In another aspect, the present invention relates to methods for predicting the efficiency of a treatment of a chronic liver fibrosis in an individual, comprising the analysis of the expression levels of at least two markers selected from the group consisting of matricellular fibrosis marker thrombospondin-2 (TSP2), insulin like growth factor binding protein 7 (IGFBP7), growth differentiation factor 15 (GDF-15), macrophage marker CD163, matricellular fibrosis marker thrombospondin-4 (TSP4), and further comprising the step of evaluating the efficacy of treatment, wherein a decrease of the expression levels of the at least two markers compared to the expression levels obtain in healthy controls or an earlier sample of said individual is indicative for an efficient treatment of the chronic liver fibrosis. In these methods of the invention, the expression levels of the markers of the marker combination are compared with the respective expression levels found in healthy control subjects or control samples.

In a further aspect, the present invention relates to a diagnostic or therapeutic test system, comprising reagents and material for detecting and/or determining the expression levels of at least two markers selected from the group consisting of matricellular fibrosis marker thrombospondin-2 (TSP2), insulin like growth factor binding protein 7 (IGFBP7), growth differentiation factor 15 (GDF-15), macrophage marker CD163, matricellular fibrosis marker thrombospondin-4 (TSP4) in a sample, and a data processing system for producing a prognostic score based on clinical variables and the expression levels obtained with said at least two markers.

The test system according to the present invention allows monitoring and diagnosing of the fibrotic progress in the individual, and therefore allows to determine the fibrogenesis of chronic liver fibrosis. For example, the marker combination of the present invention can be used to determine the time to start treatment in an individual at risk of developing a fibrotic disease, if the diagnostic test indicates that the individual is likely to develop the disease.

The expression level for the biomarkers described herein can be detected using any of a variety of known analytical methods. In one embodiment, the expression level of the biomarker of the present invention is detected using a capture reagent. In various embodiments, the capture reagent can be exposed to the biomarker in solution or can be exposed to the biomarker while the capture reagent is immobilized on a solid support. In other embodiments, the capture reagent contains a moiety that is reactive with a secondary moiety on a solid support. In these embodiments, the capture reagent can be exposed to the biomarker in solution, and then the moiety on the capture reagent can be used in conjunction with the secondary moiety on the solid support to immobilize the biomarker on the solid support. The capture reagent is preferably selected based on the type of analysis to be conducted.

Suitable capture reagents include but are not limited to aptamers, antibodies, adnectins, ankyrins, other antibody mimetics and other protein scaffolds, autoantibodies, chimeras, small molecules, Fab2 fragments, single chain antibody fragments, (single) Fv fragments, nucleic acids, lectins, ligand-binding receptors, nanobodies, imprinted polymers, peptidomimetics, receptors, and modifications and fragments of these. In some embodiments, a multiplexed format is used for the test system of the invention, in which capture reagents are immobilized, directly or indirectly, covalently or non-covalently, in discrete locations on a solid support. In some embodiments, a multiplexed format uses discrete solid supports where each solid support has a unique capture reagent associated with that solid support. In preferred embodiments, a fluorescent tag can be used to label a component of the biomarker/capture reagent complex to enable the detection of the biomarker expression level. In various embodiments, the fluorescent label can be conjugated to a capture reagent specific to any of the biomarkers described herein using known techniques, and the fluorescent label can then be used to detect the corresponding biomarker level.

In some embodiments, a test system comprises a solid support, a capture reagent, and a signal generating material. A preferred test system according to the present invention is based on enzyme-linked immunosorbent assay (ELISA). The test system is preferably used in combination with a computer system or software to analyze and report the result of the analysis of the biological sample.

The test system can also contain one or more reagents (e.g., solubilization buffers, detergents, washes, or buffers) for processing a biological sample. Examples include, but are not limited to buffers, blocking agents, mass spectrometry matrix materials, antibody capture agents, positive control samples, negative control samples, software and information such as protocols, guidance and reference data.

In some embodiments, the test systems comprise PCR primers for one or more biomarkers described herein. In some embodiments, a test system may include a DNA array containing the complement of one or more of the biomarkers described herein, reagents, and/or enzymes for amplifying or isolating sample DNA. The test systems may include reagents for real-time PCR, for example, TaqMan probes and/or primers, and enzymes.

For example, a test system may consist of a kit comprising reagents such as at least one capture reagent for determining the level of one or more biomarkers in a test sample, and optionally (b) one or more algorithms or computer programs for performing the steps of comparing the amount of each biomarker quantified in the test sample to one or more predetermined controls. In some embodiments, an algorithm or computer program assigns a score for each biomarker quantified based on said comparison and, in some embodiments, combines the assigned scores for each biomarker quantified to obtain a total score. Further, in some embodiments, an algorithm or computer program compares the total score with a predetermined score, and uses the comparison to determine whether the individual has the fibrotic disease to be determined.

The test system may include one or more devices that comprise a graphical display interface comprising interface elements such as buttons, pull down menus, scroll bars, fields for entering text, and the like as they are routinely found in graphical user interfaces known in the art. Requests entered on a user interface can be transmitted to an application program in the system for formatting to search for relevant information in one or more of the system databases.

The present invention also relates to the use of a diagnostic or therapeutic test system as described herein for diagnosis of liver fibrosis in an individual.

In another aspect, the present invention relates to a composition comprising at least two markers selected from the group consisting of matricellular fibrosis marker thrombospondin-2 (TSP2), insulin like growth factor binding protein 7 (IGFBP7), growth differentiation factor 15 (GDF-15), macrophage marker CD163, matricellular fibrosis markers thrombospondin-4 (TSP4) or ADAMTS9 for use in the treatment of chronic liver diseases by predicting the response to treatment with a candidate agent and/or monitoring the fibrogenesis of chronic liver fibrosis.

In a further aspect, the present invention furthermore relates to methods of selecting a pharmaceutical active candidate agent for inhibiting the progression of a chronic liver disease in a patient, comprising the steps of administering at least one candidate agent to said individual, and determining the expression levels of said at least two markers selected from the group consisting of matricellular fibrosis marker thrombospondin-2 (TSP2), insulin like growth factor binding protein 7 (IGFBP7), growth differentiation factor 15 (GDF-15), macrophage marker CD163, matricellular fibrosis markers thrombospondin-4 (TSP4) or ADAMTS9 in a sample obtained from said individual in the presence of said candidate agent, and comparing it to the expression levels of said at least two markers in said sample obtained from said individual in the absence of said at least one candidate agent and/or in a control sample, wherein an decrease of the expression levels of the at least two markers identifies a compound that is suitable for the prevention or treatment of chronic liver disease.

The method allows to determine pharmaceutically active candidate agents that are suitable for inhibiting the progression of a chronic liver disease in a patient, and hence are suitable for the prevention or treatment of a chronic liver disease.

The following examples are provided for illustrative purposes only and are not intended to limit the scope of the application as defined by the appended claims.

EXAMPLES

Serum/plasma samples from 248 patients with well-defined diagnosis chronic liver disease etiologies were collected. All patients underwent liver biopsy to confirm etiology, assess the degree of inflammation and especially the stage of fibrosis by expert centralized pathology and internationally used standards. Sub-cohorts of patients in follow-up of treatment, e.g., post liver transplantation (with no, rapid or super rapid fibrosis progression, or of patients with effective antiviral therapy for chronic hepatitis C) were followed for up to 12 years. In addition, survival analysis for liver transplanted patients was performed to assess the serum parameters' predictive value for patient survival. The results are shown in the following Figures. It is a key aim in biomarker development to detect patients suffering of a chronic liver disease with high sensitivity and specificity in population screening. AUROC > 0.8 defines a good, of > 0.9 a very good screening biomarker.

Description of the Figures

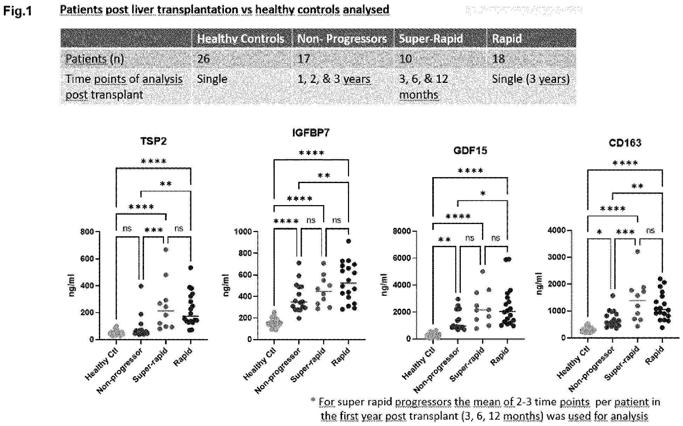

**Figure 1:** Serum levels of the fibrosis marker TSP2, the fibrosis/metabolic markers IGFBP7 and GDF15, and the innate immunity (macrophage inflammation) marker CD163 in healthy controls and in patients with liver cirrhosis due to alcoholic or viral hepatitis after liver transplantation, as determined by centralized liver biopsy and assessment by an expert liver pathologist. Comparison of patients that showed no progression, super rapid progression (within 1 year) and rapid progression (within 3 years) to histologically confirmed transplant cirrhosis. In non-progressors and super rapid progressors the means of 2-3 values taken an at the indicated time points are shown.

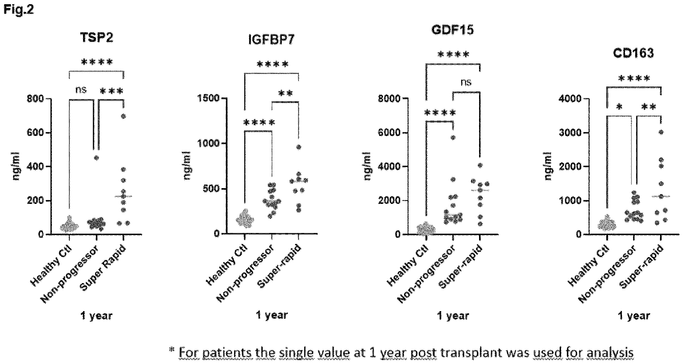

**Figure 2:** Patients as shown in Fig.1. Serum levels of the fibrosis marker TSP2, the fibrosis/metabolic markers IGFBP7 and GDF15, and the innate immunity (macrophage inflammation) marker CD163 in healthy controls and in patients with liver cirrhosis due to alcoholic of viral hepatitis after liver transplantation, as determined by centralized liver biopsy and assessment by an expert liver pathologist. Comparison of biomarker levels that were taken one year after liver transplantation comparing healthy controls, non-progressors and super rapid progressors to cirrhosis 1 year after liver transplantation.

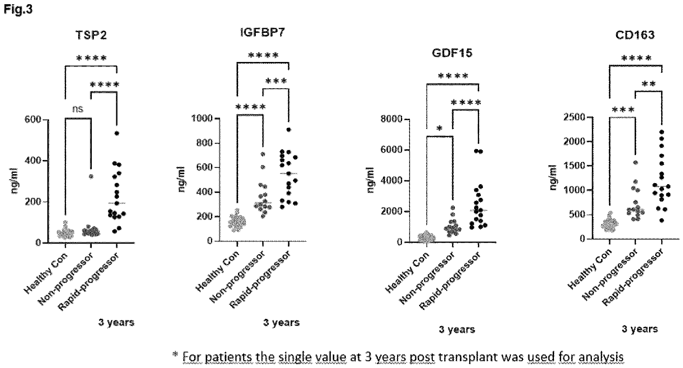

**Figure 3:** Patients as shown in Fig.1. Serum levels of the fibrosis marker TSP2, the fibrosis/metabolic markers IGFBP7 and GDF15, and the innate immunity (macrophage inflammation) marker CD163 in healthy controls and in patients with liver cirrhosis due to alcoholic of viral hepatitis after liver transplantation, as determined by centralized liver biopsy and assessment by an expert liver pathologist. Comparison of biomarker levels that were take one year after liver transplantation comparing healthy controls, non-progressors and rapid progressors 3 years after liver transplantation.

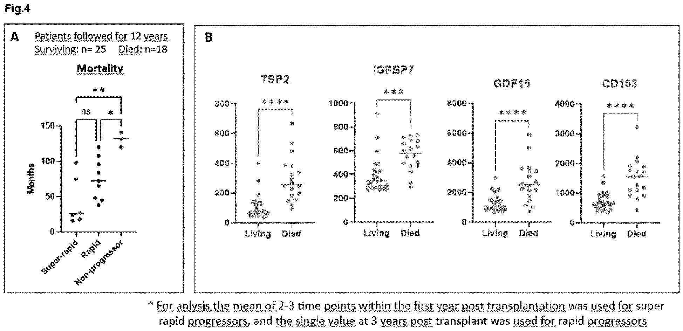

**Figure 4:** Mortality of patients defined as super rapid, rapid and non-progressors to cirrhosis after liver transplantation for alcohol related or viral liver cirrhosis, as determined by centralized liver biopsy and assessment by an expert liver pathologist with a follow-up of up to 12 years. Serum levels of the fibrosis marker TSP2, the fibrosis/metabolic markers IGFBP7 and GDF15, and the innate immunity (macrophage inflammation) marker CD163 measured 1 year after liver transplantation as early predictors of death due to rapid re-cirrhosis.

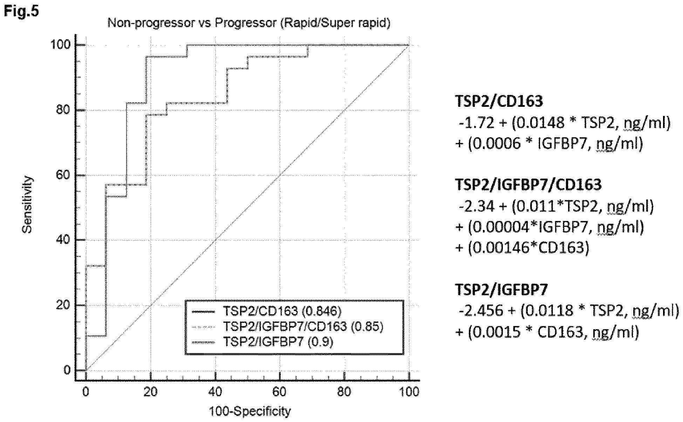

**Figure 5:** Predictive value of marker combinations determined to differentiate rapid progressors (single values at 3 years post liver transplantation) combined with super rapid progressors (means of 2-3 values per patient at 3, 6 and 12 months post transplantation) with values of non-progressors (means of 3 values at 1, 2 and between 3 years post liver transplantation, respectively) with a single determination of the serum markers.

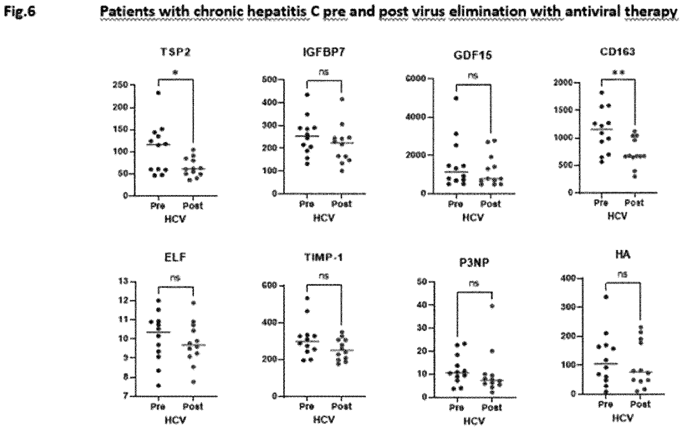

**Figure 6:** Serum levels of fibrosis (inflammation) biomarkers before and after successful elimination of hepatitis C virus with highly active antiviral therapy (duration 8-16 weeks) in patients with chronic hepatitis C and mild to moderate liver fibrosis. Significant reduction of mean values for the fibrosis markers TSP2 and the inflammation marker CD163, but not for the currently used fibrosis markers algorithm ELF (enhanced liver fibrosis test) and its constituents TIMP-1, procollagen type III aminoterminal propeptide (P3NP) and hyaluronan (HA). The majority (approx. 80%) of these patients, especially those with increased baseline marker levels will have an attenuation and even regression of their biopsy-determined liver fibrosis.

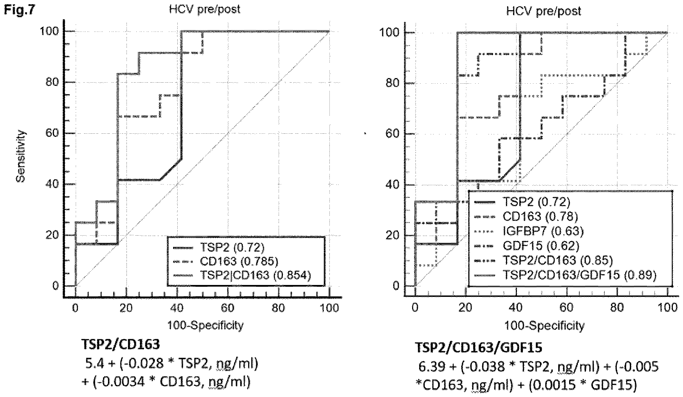

**Figure 7:** Predictive value of marker combinations comparing pre and post antiviral treatment state of liver fibrosis (fibrogenesis). Notably, the shown markers reflect the dynamics of the fibrogenic process in the liver, which is significantly attenuated with effective antiviral treatment that eliminates the primary driver of liver fibrosis, i.e., hepatitis C virus infection, in these patients.

**Figure 8:** Predictive value of combination of TSP2 levels with clinical biological (standard) laboratory parameters in 248 patients with non-alcoholic fatty liver disease (NAFLD) whose matched liver biopsies were staged and graded by an expert liver pathologist according to international criteria. Patients with the non-alcoholic steatohepatitis (NASH; i.e., a NAS score $\geq$ 4) and significant liver fibrosis (stage $\geq$ 2) are at a high risk to progress to cirrhosis (NASH at risk). It is a key aim in biomarker development to detect these patients with high sensitivity and specificity in population screening. An AUROC > 0.8 defines a good, of > 0.9 a very good screening biomarker.

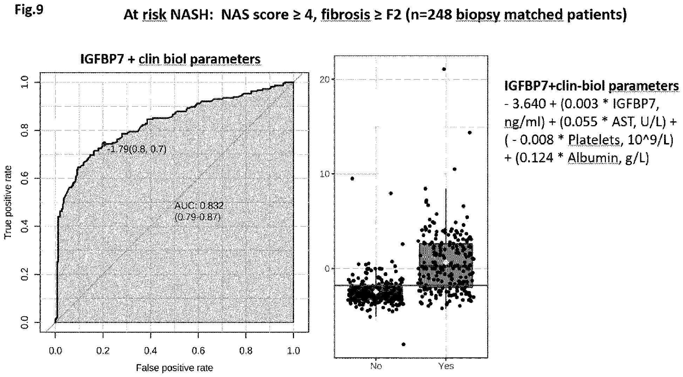

**Figure 9:** Predictive value of combination of IGFBP7 levels with clinical biological (standard) laboratory parameters in 248 patients with non-alcoholic fatty liver disease (NAFLD) whose matched liver biopsies were staged and graded by an expert liver pathologist according to international criteria. Patients with the non-alcoholic steatohepatitis (NASH; i.e., a NAS score $\geq$ 4) and significant liver fibrosis (stage $\geq$ 2) are at a high risk to progress to cirrhosis (NASH at risk). It is a key aim in biomarker development to detect these patients with high sensitivity and specificity in population screening. An AUROC > 0.8 defines a good, of > 0.9 a very good screening biomarker.

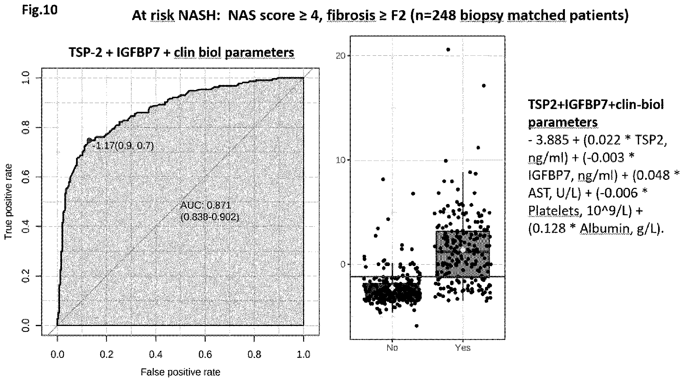

**Figure 10:** Predictive value of combination of TSP2, IGFBP7 levels with clinical biological (standard) laboratory parameters in 248 patients with non-alcoholic fatty liver disease (NAFLD) whose matched liver biopsies were staged and graded by an expert liver pathologist according to international criteria. Patients with the non-alcoholic steatohepatitis (NASH; i.e., a NAS score $\geq$ 4) and significant liver fibrosis (stage $\geq$ 2) are at a high risk to progress to cirrhosis (NASH at risk). It is a key aim in biomarker development to detect these patients with high sensitivity and specificity in population screening. An AUROC > 0.8 defines a good, of > 0.9 a very good screening biomarker.

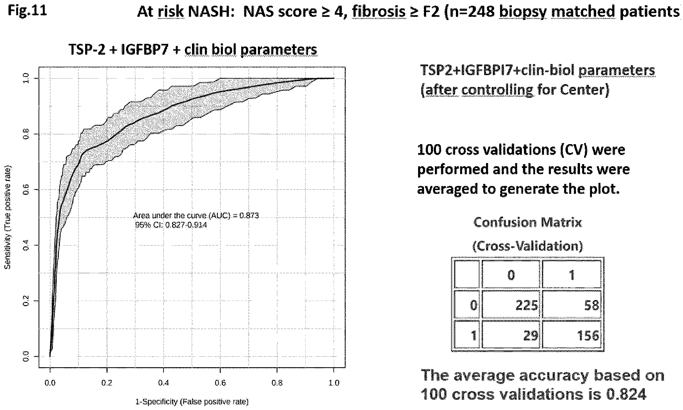

**Figure 11:** Predictive value of combination of Tsp2 and IGFBP7 levels with clinical biological (standard) laboratory parameters in 248 patients with non-alcoholic fatty liver disease (NAFLD) whose matched liver biopsies were staged and graded by an expert liver pathologist according to international criteria. Extensive cross validation of the data as detailed in Methods. Patients with the non-alcoholic steatohepatitis (NASH; i.e., a NAS score $\geq$ 4) and significant liver fibrosis (stage $\geq$ 2) are at a high risk to progress to cirrhosis (NASH at risk). It is a key aim in biomarker development to detect these patients with high sensitivity and specificity in population screening.

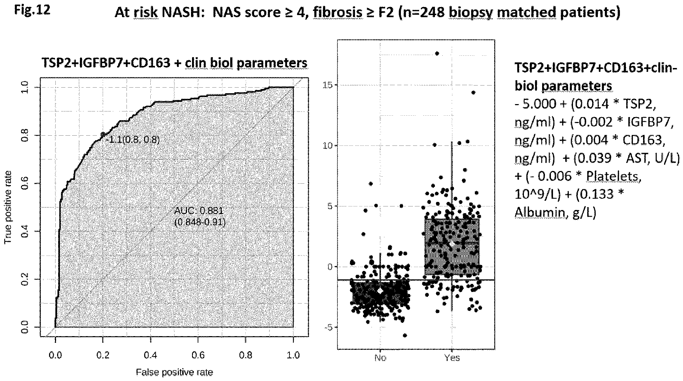

**Figure 12:** Predictive value of combination of Tsp2, IGFBP7 and CD163 levels with clinical biological (standard) laboratory parameters in 248 patients with non-alcoholic fatty liver disease (NAFLD) whose matched liver biopsies were staged and graded by an expert liver pathologist according to international criteria. Patients with the non-alcoholic steatohepatitis (NASH; i.e., a NAS score $\geq$ 4) and significant liver fibrosis (stage $\geq$ 2) are at a high risk to progress to cirrhosis (NASH at risk). It is a key aim in biomarker development to detect these patients with high sensitivity and specificity in population screening. An AUROC > 0.8 defines a good, of > 0.9 a very good screening biomarker.

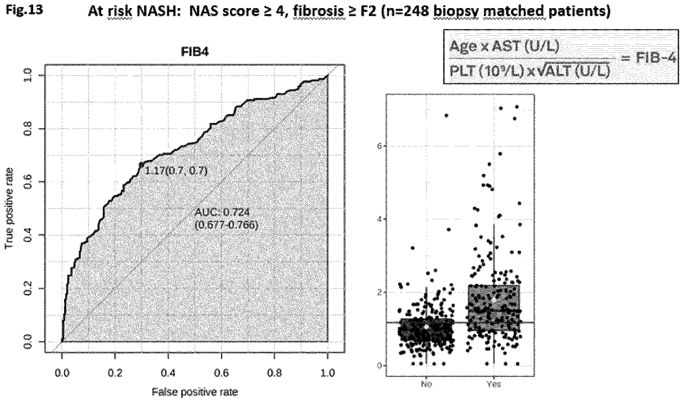

**Figure 13:** Predictive value of currently widely used FIB-4 score, based on clinical laboratory parameters (age; AST, aspartate aminotransferase; PLT, platelets) in 248 patients with non-alcoholic fatty liver disease (NAFLD) whose matched liver biopsies were staged by an expert liver pathologist and graded according to international criteria. This score which serves as a general comparator across different studies has a much low predictive value (AUROC 0.724) for NASH at risk. Patients with the non-alcoholic steatohepatitis (NASH; i.e., a NAS score $\geq$ 4) and significant liver fibrosis (stage $\geq$ 2) are at a high risk to progress to cirrhosis (NASH at risk). It is a key aim in biomarker development to detect these patients with high sensitivity and specificity in population screening.

**Figure 14:** Predictive value of the combination of TSP2, IGFBP7 and CD163 levels in 248 patients with non-alcoholic fatty liver disease (NAFLD) whose matched liver biopsies were staged by an expert liver pathologist according to international criteria. Regulatory authorities consider patients fibrosis F $\geq$ 2 at high risk to develop cirrhosis, and therefore would be candidates for antifibrotic therapies. Here, the pure fibrosis marker Tsp2 is less strikingly improved by addition of the fibrosis/metabolic marker IGFBP7 and the inflammatory marker CD163. AUROC > 0.8 defines a good, of > 0.9 a very good screening biomarker.

**Figure 15:** Predictive value of currently widely used FIB-4 score, based on age and clinical laboratory parameters in 248 patients with non-alcoholic fatty liver disease (NAFLD) whose matched liver biopsies were centrally staged for fibrosis by an expert liver pathologist according to international criteria. This score which serves as a general comparator across different studies has a lower predictive value (AUROC 0.727) for fibrosis stage F $\geq$ 2 than the algorithm in Fig.14.

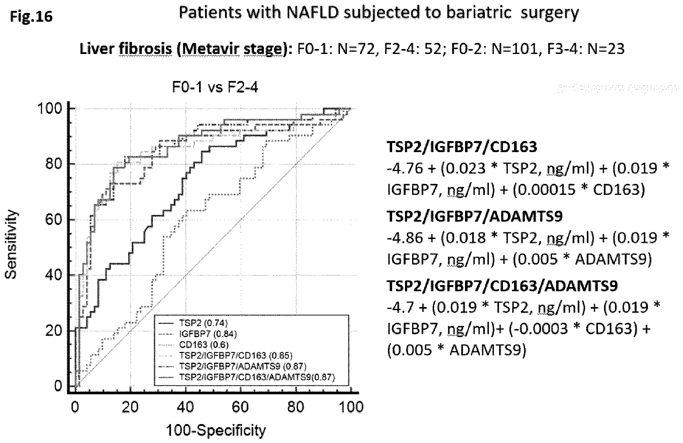

**Figure 16:** Predictive value of the combination of TSP2, IGFBP7 and CD163 levels to predict fibrosis F $\geq$ 2 in 124 patients before they underwent bariatric surgery for severe obesity (body mass index > 45) and prediabetes/diabetes type 2, and various degrees of liver inflammation due to non-alcoholic fatty liver disease (NAFLD) whose matched liver biopsies were staged by an expert liver pathologist according to international criteria (samples from Dr. K. Clement, Paris). Here, the pure fibrosis marker TSP2 performs less well, but its prediction for fibrosis is significantly improved in combinations, especially with the metabolic/fibrosis marker IGFBP7. Another, largely type 2 diabetes related marker, ADAMTS9 (a disintegrin and metalloproteinase, thrombospondin (domain protein) 9; proprietary assay) further improves fibrosis prediction in this special NAFLD cohort. AUROC > 0.8 defines a good, of > 0.9 a very good screening biomarker.

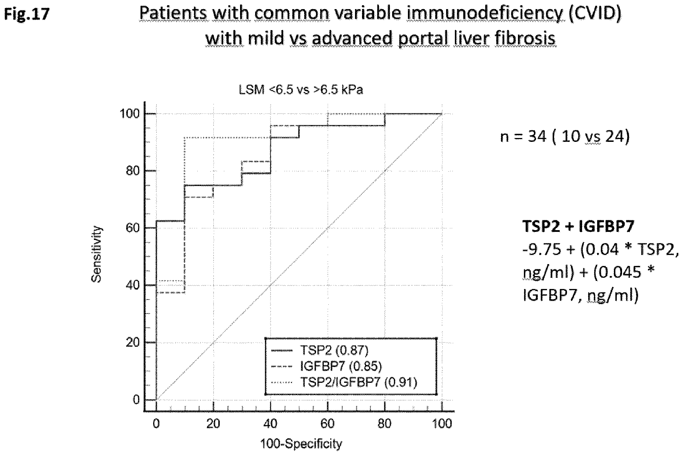

**Figure 17:** Predictive value of the combination of TSP2 and IGFBP7 to diagnose fibrosis F $\geq$ 3 in 34 patients with common variable immunodeficiency (CVID). Up to 30 % of CVID patients develop a portal (granulomatous) fibrosis that can rapidly proceed to cirrhosis. Biopsy sampling error for fibrosis staging is high. Therefore, transient elastography (liver stiffness measurement, LSM) is currently the best method to detect pre-cirrhosis and cirrhosis (stages F3 and F4, respectively), as is used as reference parameter in this cohort. The combination of TSP2 and IGFB7 has a unique predictive value (AUROC 0.91) to diagnose advanced fibrosis.

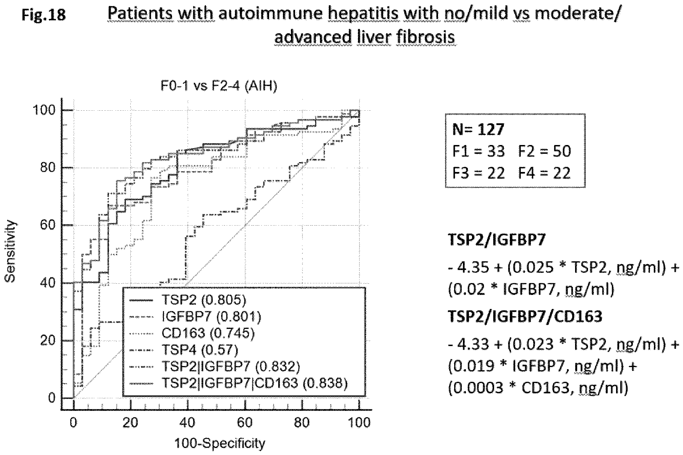

**Figure 18:** Predictive value of the combination of TSP2, IGFBP7 and CD163 to diagnose significant fibrosis F ≥ 2 vs mild fibrosis F=1, as determined by centralized liver biopsy and assessment by an expert liver pathologist in 127 patients with autoimmune hepatitis at diagnosis. Thrombbospondind-4 (TSP4) is a matricellular protein related to TSP2, being related to epithelial-mesenchymal transition (EMT), a driver of biliary fibrosis. TSP4 does not contribute to fibrosis prediction in this disease that does not show prominent EMT. If left untreated (with immune suppressant drugs), most of these patients develop advanced fibrosis/cirrhosis within 5-10 years. The combination of TSP2, IGFB7 and CD163 has the best predictive value (AUROC 0.838) to diagnose significant fibrosis.

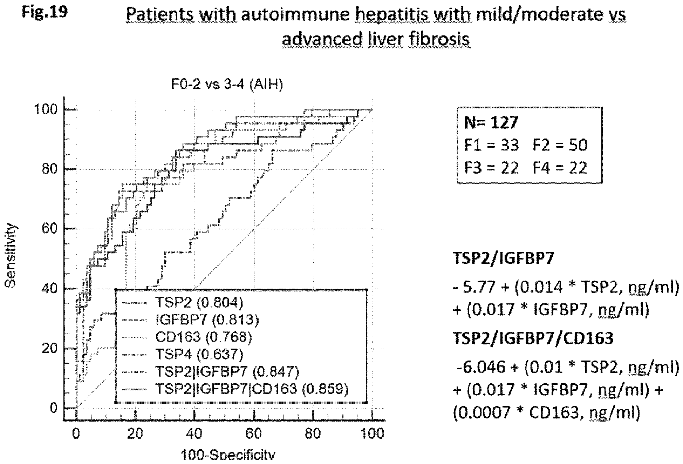

**Figure 19:** Predictive value of the combination of TSP2, IGFBP7 and CD163 to diagnose advanced fibrosis/cirrhosis F ≥ 3 vs mild to moderate fibrosis F1-F2, as determined by centralized liver biopsy and assessment by an expert liver pathologist in 127 patients with autoimmune hepatitis at diagnosis. Thrombospondin-4 (TSP4) is a matricellular protein related to TSP2, being involved in epithelial-mesenchymal transition (EMT), a driver of biliary fibrosis. TSP4 does not contribute to fibrosis prediction in this disease that does not show prominent EMT. If left untreated (with immune suppressant drugs), most of these patients develop advanced fibrosis/cirrhosis within 5-10 years. The combination of TSP2, IGFB7 and CD163 has the best predictive value (AUROC 0.838) to diagnose advanced fibrosis (F3) or cirrhosis (F4).

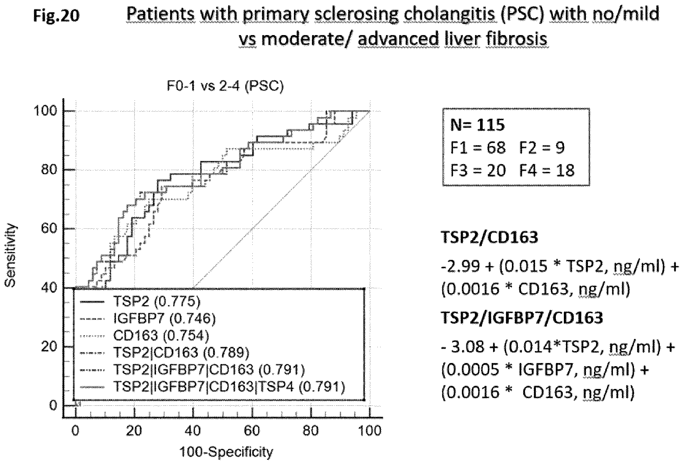

**Figure 20:** Predictive value of the combination of TSP2, IGFBP7, CD163 and TSP4 to diagnose significant fibrosis F ≥ 2 vs mild fibrosis F=1 as determined by centralized liver biopsy assessment by an expert liver pathologist and liver stiffness measurement (LSM) (cut-off 16.3 kPa for cirrhosis) in 115 patients with primary sclerosing cholangitis (PSC). Thrombospondin-4 (TSP4) is a matricellular protein related to TSP2, being involved in epithelial-mesenchymal transition (EMT), a driver of biliary fibrosis. TSP4 contributes to fibrosis prediction in this disease that shows EMT by fibrogenic cholangiocytes. To date there is no drug effective drug treatment for PSC, and most of these patients develop cirrhosis within 10 years. The combination of TSP2, IGFB7 and CD163 (and TSP4) has the best predictive value (AUROC 0.791) to diagnose significant fibrosis, which in PSC is difficult to assess with biopsy due to a high biopsy sampling error.

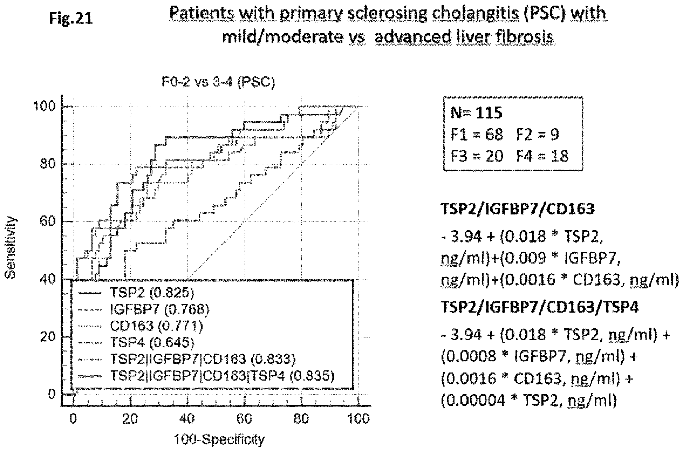

**Figure 21:** Predictive value of the combination of TSP2, IGFBP7, CD163 and TSP4 to diagnose advanced fibrosis (F3) and cirrhosis (F4) vs mild to significant fibrosis F1-F2), as determined by centralized liver biopsy assessment by an expert liver pathologist and liver stiffness measurement (LSM) (cut-off 16.3 kPa for cirrhosis) in 115 patients with primary sclerosing cholangitis (PSC). Thrombospondin-4 (TSP4) that is involved in epithelial-mesenchymal transition (EMT which contributes to fibrosis prediction in PSC via fibrogenic cholangiocytes. There is no effective drug treatment for PSC, and most patients develop cirrhosis within 10 years. The combination of TSP2, IGFB7 and CD163 (and TSP4) has the best predictive value (AUROC 0.835) to diagnose significant fibrosis, which in PSC is difficult to assess with biopsy due to a high biopsy sampling error.

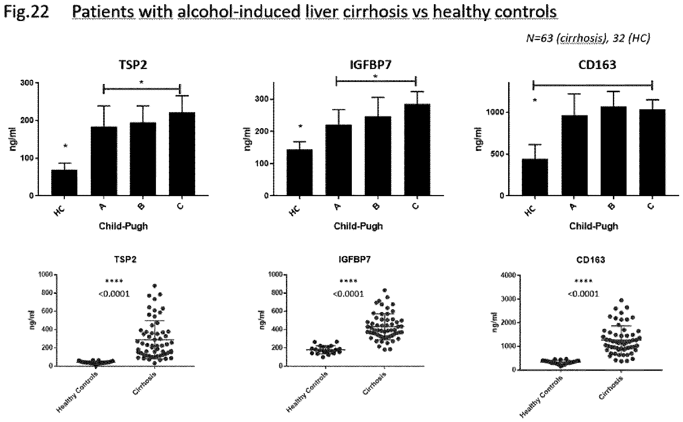

**Figure 22:** Predictive value of TSP2, IGFBP7 and CD163 to diagnose alcohol induced liver cirrhosis (F=4) of Child-Pugh class A (compensated, often undetected), class B (partly decompensated), and C (fully decompensated) vs healthy controls (HC) in 63 patients subject to portal-central vein catheterization and pressure gradient determination. Predictive value of markers does not differ between Child-Pugh classes A-C, which can be explained by the independent role of fibrosis vs mere parameters of liver inflammation and function. Moreover, the parameters are dynamic and reflect fibrogenesis, i.e., synthesis/deposition and turnover of the fibrotic extracellular matrix (ECM).

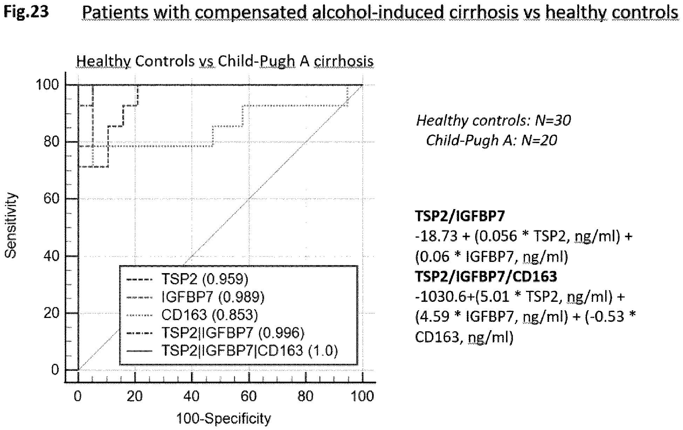

**Figure 23:** High predictive value of combined TSP2, IGFBP7 and CD163 to differentiate compensated alcohol induced liver cirrhosis (Child-Pugh A) from healthy controls. Notably, patients with compensated cirrhosis usually are clinically unapparent, while they may have risk factors, such as esophageal varices or a higher risk for infection, sudden decompensation, or the development of primary liver cancer. Routine laboratory values are often normal.

## Methods and Materials

## Patient selection, samples, and study groups

## Determination of plasma GDF15, CD163, TSP2 and IGFBP7 by enzyme linked immunosorbent assay (ELISA)

Plasma GDF15 and CD163, levels were measured according to the manufacturer's instructions using DuoSet ELISAs from R&D systems with altered antibody combinations to increase sensitivity and specificity. Assay linearity allowed reliable measurements between 62.5 to 1000 pg/ml for GDF15 and 325 to 20,000 pg/ml for CD163. Normal plasma levels were confirmed with samples of the inventors' own plasma repository, and inter- and intra- assay variabilities below 15 % were reached using 5 normal and 5 pathological plasmas in 6 replicates. Plasma matricellular markers thrombospondin-2 (TSP2) and insulin like growth factor binding protein 7 (IGFBP7) were measured using in-house developed ELISAs. Briefly, 96 well plates were coated with 100 ng/well of monoclonal antibodies from LSBio (LS-C132440) and Thermofischer (MA5-30520). Plates were incubated overnight at 4°C followed by washing with PBS/0.1% Tween-20 and blocking with 1% BSA for 1 hr at RT. After a second wash, samples along with standards (in-house recombinant TSP2 and IGFBP7 and blank controls) were added to the wells, incubated for 2 hrs at RT followed by washing. Goat polyclonal anti-TSP2 and anti-IGFBP7 antibodies purchased from R&D systems (AF1635 and AF1334) were labeled with horseradish peroxidase (HRP) using an HRP conjugation kit (Abcam, ab102890). Predetermined HRP-antibody dilutions were added to the plates for 1 hr, followed by washing and addition of 100 $\mu$l ready-to-use tetramethyl-benzidine (Thermofisher; Cat: N301) for 15 minutes at RT in the dark. The reaction was stopped with 50 $\mu$l 1% HCL, and OD was measured at 450 nm with 570 nm as reference. The linear range was between 162 to 10,000 pg/ml for TSP2 and 325 to 5,000 pg/ml for IGFBP7. Both assays were highly reliable, and intra- and inter-assay variabilities were below 10% and 13%, respectively.

**Statistics and calculation of biomarker algorithms**

One-way ANOVA test was performed using the Graph Pad Prism 9 and the logistic regression analysis was performed in MedCalc statistical software using backward stepwise ratio model including TSP2, CD163 and GDF15. Examples are given for NAFLD/NASH liver fibrosis, which can be calculated as follows:

$$\text{logit}(p) = -7.3 + 0.02*TSP2 + 0.004*GDF15 + 0.01*CD163; \text{logit}(p) = -10.65 + (-0.05)*TSP2 + 0.007*GDF15 + 0.0176*CD163 \ \& \ \text{logit}(p) = -2.5 + 0.005*TSP2 + 0.0005*GDF15 + 0.002*CD163.$$

Alternative formulas in context with other liver disease etiologies are given in the accompanying Figures and Tables.

**Combinatorial score**

A combinatorial prognostic score was used, incorporating clinical variables and/or multi-biomarkers, through a comprehensive multivariable analysis employing logistic regression. This logistic regression is constructed using a backward stepwise approach, aimed at simplifying the model by iteratively eliminating variables that lack statistical significance or do not substantially enhance the model's predictive capability. The prognostic score is computed as a weighted sum, following the formula below:

**At - Risk NASH**

TSP2_clin_biol_parameters: - 3.792 + (0.019 * TSP2, ng/ml) + (0.043 * AST, U/L) + (- 0.006 * Platelets,$10^9$/L) + (0.125 * Albumin, g/L) + (- 0.002 * GGT, U/L).

I7_clin_biol parameters: - 3.640 + (0.003 * IGFBP7, ng/ml) + (0.055 * AST. U/L) + (- 0.008 * Platelets,$10^9$/L) + (0.124 * Albumin, g/L).

TSP2_I7 clin biol parameters; - 3.885 + (0.022 * TSP2, ng/ml) + (-0.003 * IGFBP7, ng/ml) + (0.048 * AST, U/L) + (- 0.006 * Platelets, $10^9$/L) + (0.128 * Albumin, g/L).

TSP2_IGFBP7_CD163_clin_biol parameters; - 5.000 + (0.014 * TSP2, ng/ml) + (-0.002 * IGFBP7, ng/ml) + (0.004 * CD163, ng/ml) + (0.039 * AST, U/L) + (- 0.006 * Platelets, $10^9$/L) + (0.133 * Albumin, g/L).

**Advanced Fibrosis**

TSP2_clin_biol parameters: - 1.836 + (0.023 * TSP2, ng/ml) + (0.034 * Age) + (- 0.006 * Platelets, $10^9$/L).

IGFBP7_clin_biol parameters; - 2.142 + (0.005 * IGFBP7, ng/ml) + (0.028 * Age) + (- 0.006 * Platelets, $10^9$/L).

TSP2_IGFBP7 clin biol parameters: - 1.839 + (0.025 * TSP2, ng/ml) + (-0.002 * IGFBP7, ng/ml) + (0.037 * AgeE) + (- 0.006 * Platelets $10^9$/L).

TSP2_IGFBP7_CD163_clin_biol parameters: = - 2.685 + (0.020 * TSP2, ng/ml) + (-0.002 * IGFBP7, ng/ml) + (0.003 * CD163 ng/ml) + (0.029 * Age) + (- 0.006 * Platelets $10^9$L).

The multivariable logistic models were accounted for center effects, age, BMI, and gender. For prediction, both classical and advanced Machine Learning algorithms were applied, assessing their predictive performance through AUROC analysis.

**Claims**

1. A method for detecting the presence and/or predicting the severity or stage of chronic liver fibrosis in an individual, comprising the steps of (a) determining the expression levels of at least two markers selected from the group consisting of matricellular fibrosis marker thrombospondin-2 (TSP2), insulin like growth factor binding protein 7 (IGFBP7), growth differentiation factor 15 (GDF15), macrophage marker CD163, matricellular fibrosis markers thrombospondin-4 (TSP4) or ADAMTS9 in a biological sample obtained from said individual, (b) comparing the expression levels of said at least two markers in said biological sample with the expression levels of said at least two markers in a sample obtained from a healthy control or a control sample, wherein an increase of the expression of said at least two markers in said individual compared to the healthy control or control sample is indicative for the presence, severity and/or stage of chronic liver fibrosis in said individual.

2. The method for according to claim 1, wherein the determination of the expression levels is carried out with marker DNA, RNA or protein, and said biological sample is derived from blood serum or plasma, other body fluids, or biopsy material obtained from said individual.

3. The method according to claim 1, wherein said chronic liver fibrosis relates to a liver disease selected from the group consisting of non-alcoholic steatohepatitis (NAFLD/NASH), alcoholic liver disease (ALD), alcoholic hepatitis (AH), viral hepatitis B, C or D, post-transplant liver disease, common variable immunodeficiency (CVID), autoimmune hepatitis (AIH), genetic liver disease, primary biliary cholangitis (PBC), primary sclerosing cholangitis (PSC), or chronic infectious liver disease, e.g., schistosomiasis.

4. The method according to any one of claims 1 to 3, wherein a prognostic score for each of the liver disease etiologies is determined by incorporating clinical biological parameters and values of the determined expression levels of said at least two markers by applying a comprehensive multivariable analysis employing logistic regression.

5. The method according to claim 4, wherein said logistic regression is constructed using a backward stepwise approach by iteratively eliminating variables that lack statistical significance or do not substantially enhance the model's predictive capability.

6. The method according to claim 4, wherein the AUROC (area under receiver operating characteristics) is > 0.8.

7. The method according to any one of claims 1 to 6, wherein at least two markers of the following marker combinations are selected for the respective liver disease etiology:

| Disease | TSP2 | IGFBP7 | GDF-15 | CD163 | TSP4 |
|---|---|---|---|---|---|
| NAFLD/NASH | X | X | | X | |
| ALD/AH | X | X | | X | |
| Hepatitis C | X | X | X | X | |
| Post transplant | X | X | X | X | |
| AIH | X | X | | X | |
| CVID | X | X | | | |
| PBC / PSC | X | X | | X | X |

8. The method according to claim 7, wherein in addition to the at least two markers of the marker combinations the marker ADAMTS9 is determined, in particular in type 2 diabetes individuals undergoing bariatric surgery or being overweight or morbidly obese.

9. The method according to any one of claims 1 to 8, wherein the marker combination consisting of TSP2, IGFBP7 and CD163 is utilized for comparing their expression levels to the expression levels of a sample obtained from a healthy control or a control sample.

10. A method for predicting the efficacy of a treatment of a chronic liver fibrosis in an individual, comprising the analysis of at least two markers selected from the group consisting of matricellular fibrosis marker thrombospondin-2 (TSP2), insulin like growth factor binding protein 7 (IGFBP7), growth differentiation factor 15 (GDF15), macrophage marker CD163, matricellular fibrosis markers thrombospondin-4 (TSP4) or ADAMTS9 according to a method of any one of claims 1 to 9, and further comprising the step of evaluating the efficacy of treatment, wherein a decrease of the expression levels of the at least two markers compared to the expression levels obtain in healthy controls or an earlier sample of said individual is indicative for an efficient treatment of the chronic liver fibrosis.

11. A diagnostic or therapeutic test system, comprising reagents and material for detecting and/or determining the expression levels of at least two markers selected from the group consisting of matricellular fibrosis marker thrombospondin-2 (TSP2), insulin like growth factor binding protein 7 (IGFBP7), growth differentiation factor 15 (GDF15), macrophage marker CD163, matricellular fibrosis markers thrombospondin-4 (TSP4) or ADAMTS9in a sample, and a data processing system for producing a prognostic score based on clinical biological parameters and expression levels determined with the at least two markers.

12. Use of a diagnostic or therapeutic test system according to claim 11 for diagnosis of liver fibrosis in an individual.

13. A composition comprising at least two markers selected from the group consisting of matricellular fibrosis marker thrombospondin-2 (TSP2), insulin like growth factor binding protein 7 (IGFBP7), growth differentiation factor 15 (GDF15), macrophage marker CD163, matricellular fibrosis markers thrombospondin-4 (TSP4) or ADAMTS9 for use in the treatment of chronic liver diseases by predicting the response to treatment with a candidate agent, nutritional or life style interventions, and/or monitoring the fibrogenesis of chronic liver fibrosis.

14. A method of selecting a pharmaceutically active candidate agent, or a nutritional or life style intervention for inhibiting the progression of a chronic liver disease in a patient, comprising the steps of administering at least one candidate agent or measure to said individual, and determining the expression levels of said at least two markers selected from the group consisting of matricellular fibrosis marker thrombospondin-2 (TSP2), insulin like growth factor binding protein 7 (IGFBP7), growth differentiation factor 15 (GDF15), macrophage marker CD163, matricellular fibrosis markers thrombospondin-4 (TSP4) or ADAMTS9 in a sample obtained from said individual in the presence of said candidate agent or mentioned intervention, and comparing it to the expression levels of said at least two markers in said sample obtained from said individual in the absence of said at least one candidate agent/ intervention and/or in a control sample, wherein an decrease of the expression levels of the at least two markers identifies a compound that is suitable for the prevention or treatment of chronic liver disease.

15. A pharmaceutically active candidate agent or other therapeutic intervention derived by a method according to claim 14.

**Fig.1** <u>Patients post liver transplantation vs healthy controls analysed</u>

| | Healthy Controls | Non- Progressors | Super-Rapid | Rapid |
|---|---|---|---|---|
| Patients (n) | 26 | 17 | 10 | 18 |
| Time points of analysis post transplant | Single | 1, 2, & 3 years | 3, 6, & 12 months | Single (3 years) |

TSP2  IGFBP7  GDF15  CD163

\* For super rapid progressors the mean of 2-3 time points per patient in the first year post transplant (3, 6, 12 months) was used for analysis

Fig.2

TSP2

IGFBP7

GDF15

CD163

1 year

1 year

1 year

1 year

* For patients the single value at 1 year post transplant was used for analysis

Fig.3

TSP2

IGFBP7

GDF15

CD163

* For patients the single value at 3 years post transplant was used for analysis

**Fig.4**

* For anlysis the mean of 2-3 time points within the first year post transplantation was used for super rapid progressors, and the single value at 3 years post transplant was used for rapid progressors

**Fig.5**

Non-progressor vs Progressor (Rapid/Super rapid)

Legend:
- TSP2/CD163 (0.846)
- TSP2/IGFBP7/CD163 (0.85)
- TSP2/IGFBP7 (0.9)

**TSP2/CD163**
-1.72 + (0.0148 * TSP2, ng/ml)
+ (0.0006 * IGFBP7, ng/ml)

**TSP2/IGFBP7/CD163**
-2.34 + (0.011*TSP2, ng/ml)
+ (0.00004*IGFBP7, ng/ml)
+ (0.00146*CD163)

**TSP2/IGFBP7**
-2.456 + (0.0118 * TSP2, ng/ml)
+ (0.0015 * CD163, ng/ml)

**Fig.6** <u>Patients with chronic hepatitis C pre and post virus elimination with antiviral therapy</u>

**Fig.7**

HCV pre/post

HCV pre/post

**TSP2/CD163**
5.4 + (-0.028 * TSP2, ng/ml)
+ (-0.0034 * CD163, ng/ml)

**TSP2/CD163/GDF15**
6.39 + (-0.038 * TSP2, ng/ml) + (-0.005
*CD163, ng/ml) + (0.0015 * GDF15)

**Fig.8**

**At risk NASH:  NAS score ≥ 4, fibrosis ≥ F2 (n=248 biopsy matched patients)**

**TSP2 + clin biol parameters**

**TSP2+clin-biol-parameters**

- 3.792 + (0.019 * TSP2, ng/ml) + (0.043 * AST, U/L) + ( - 0.006 * Platelets 10^9/L) + (0.125 * Albumin, g/L) + (- 0.002 * GGT, U/L)

Fig.9   **At risk NASH:  NAS score ≥ 4, fibrosis ≥ F2 (n=248 biopsy matched patients)**

**IGFBP7 + clin biol parameters**

**IGFBP7+clin-biol parameters**
- 3.640 + (0.003 * IGFBP7, ng/ml) + (0.055 * AST, U/L) + ( - 0.008 * Platelets, 10^9/L) + (0.124 * Albumin, g/L)

Fig.10        **At risk NASH: NAS score ≥ 4, fibrosis ≥ F2 (n=248 biopsy matched patients)**

**TSP-2 + IGFBP7 + clin biol parameters**

AUC: 0.871
(0.838-0.902)

-1.17(0.9, 0.7)

True positive rate

False positive rate

**TSP2+IGFBP7+clin-biol parameters**
- 3.885 + (0.022 * TSP2, ng/ml) + (-0.003 * IGFBP7, ng/ml) + (0.048 * AST, U/L) + (-0.006 * Platelets, 10^9/L) + (0.128 * Albumin, g/L).

**Fig.11**     **At risk NASH:  NAS score ≥ 4, fibrosis ≥ F2 (n=248 biopsy matched patients**

### TSP-2 + IGFBP7 + clin biol parameters

Area under the curve (AUC) = 0.873
95% CI: 0.827-0.914

TSP2+IGFBPI7+clin-biol parameters
(after controlling for Center)

**100 cross validations (CV) were performed and the results were averaged to generate the plot.**

Confusion Matrix

(Cross-Validation)

|   | 0 | 1 |
|---|---|---|
| 0 | 225 | 58 |
| 1 | 29 | 156 |

The average accuracy based on 100 cross validations is 0.824

**Fig.12**        **At risk NASH:  NAS score ≥ 4, fibrosis ≥ F2 (n=248 biopsy matched patients)**

**TSP2+IGFBP7+CD163 + clin biol parameters**

AUC: 0.881
(0.848-0.91)

1.1(0.8, 0.8)

True positive rate

False positive rate

TSP2+IGFBP7+CD163+clin-
biol parameters
- 5.000 + (0.014 * TSP2,
ng/ml) + (-0.002 * IGFBP7,
ng/ml) + (0.004 * CD163,
ng/ml)  + (0.039 * AST, U/L)
+ (- 0.006 * Platelets,
10^9/L) + (0.133 *
Albumin, g/L)

Fig.13    At risk NASH:  NAS score ≥ 4, fibrosis ≥ F2 (n=248 biopsy matched patients)

**FIB4**

1.17(0.7, 0.7)

AUC: 0.724
(0.677-0.766)

True positive rate

False positive rate

$$\frac{\text{Age} \times \text{AST (U/L)}}{\text{PLT (10}^9\text{/L)} \times \sqrt{\text{ALT (U/L)}}} = \text{FIB-4}$$

No    Yes

**Fig.14** <u>Liver fibrosis ≥ F2 (n=248 biopsy matched patients with NAFLD)</u>

**TSP2+IGFBP7+CD163 + clin biol parameters**

**TSP2+ IGFBP7 + CD163 + clin biol parameters**
- 2.685 + (0.020 * TSP2, ng/ml) + (-0.002 * IGFBP7, ng/ml) + (0.003 * CD163, ng/ml) + (0.029 * Age) + (- 0.006 * Platelets, 10^9/L).

**Fig.15** <u>Liver fibrosis ≥ F2 (n=248 biopsy matched patients with NAFLD)</u>

$$\frac{\text{Age} \times \text{AST (U/L)}}{\text{PLT} (10^9/\text{L}) \times \sqrt{\text{ALT (U/L)}}} = \text{FIB-4}$$

**Fig.16**  Patients with NAFLD subjected to bariatric surgery

**Liver fibrosis (Metavir stage):** F0-1: N=72, F2-4: 52; F0-2: N=101, F3-4: N=23

F0-1 vs F2-4

Legend:
- TSP2 (0.74)
- IGFBP7 (0.84)
- CD163 (0.6)
- TSP2/IGFBP7/CD163 (0.85)
- TSP2/IGFBP7/ADAMTS9 (0.87)
- TSP2/IGFBP7/CD163/ADAMTS9 (0.87)

**TSP2/IGFBP7/CD163**
-4.76 + (0.023 * TSP2, ng/ml) + (0.019 * IGFBP7, ng/ml) + (0.00015 * CD163)

**TSP2/IGFBP7/ADAMTS9**
-4.86 + (0.018 * TSP2, ng/ml) + (0.019 * IGFBP7, ng/ml) + (0.005 * ADAMTS9)

**TSP2/IGFBP7/CD163/ADAMTS9**
-4.7 + (0.019 * TSP2, ng/ml) + (0.019 * IGFBP7, ng/ml)+ (-0.0003 * CD163) + (0.005 * ADAMTS9)

**Fig.17**  <u>Patients with common variable immunodeficiency (CVID) with mild vs advanced portal liver fibrosis</u>

LSM <6.5 vs >6.5 kPa

Legend:
- TSP2 (0.87)
- IGFBP7 (0.85)
- TSP2/IGFBP7 (0.91)

Y-axis: Sensitivity
X-axis: 100-Specificity

n = 34 ( 10 vs 24)

**TSP2 + IGFBP7**
-9.75 + (0.04 * TSP2, ng/ml) + (0.045 * IGFBP7, ng/ml)

**Fig.18** <u>Patients with autoimmune hepatitis with no/mild vs moderate/</u>
<u>advanced liver fibrosis</u>

F0-1 vs F2-4 (AIH)

Legend:
— TSP2 (0.805)
--- IGFBP7 (0.801)
····· CD163 (0.745)
-·-·- TSP4 (0.57)
-··-·· TSP2|IGFBP7 (0.832)
—— TSP2|IGFBP7|CD163 (0.838)

N= 127
F1 = 33   F2 = 50
F3 = 22   F4 = 22

**TSP2/IGFBP7**

- 4.35 + (0.025 * TSP2, ng/ml) +
(0.02 * IGFBP7, ng/ml)

**TSP2/IGFBP7/CD163**

- 4.33 + (0.023 * TSP2, ng/ml) +
(0.019 * IGFBP7, ng/ml) +
(0.0003 * CD163, ng/ml)

**Fig.19** Patients with autoimmune hepatitis with mild/moderate vs advanced liver fibrosis

F0-2 vs 3-4 (AIH)

Legend:
- TSP2 (0.804)
- IGFBP7 (0.813)
- CD163 (0.768)
- TSP4 (0.637)
- TSP2|IGFBP7 (0.847)
- TSP2|IGFBP7|CD163 (0.859)

N= 127
F1 = 33   F2 = 50
F3 = 22   F4 = 22

**TSP2/IGFBP7**

- 5.77 + (0.014 * TSP2, ng/ml)
+ (0.017 * IGFBP7, ng/ml)

**TSP2/IGFBP7/CD163**

-6.046 + (0.01 * TSP2, ng/ml)
+ (0.017 * IGFBP7, ng/ml) +
(0.0007 * CD163, ng/ml)

**Fig.20** <u>Patients with primary sclerosing cholangitis (PSC) with no/mild vs moderate/ advanced liver fibrosis</u>

F0-1 vs 2-4 (PSC)

TSP2 (0.775)
IGFBP7 (0.746)
CD163 (0.754)
TSP2|CD163 (0.789)
TSP2|IGFBP7|CD163 (0.791)
TSP2|IGFBP7|CD163|TSP4 (0.791)

N= 115
F1 = 68  F2 = 9
F3 = 20  F4 = 18

**TSP2/CD163**

-2.99 + (0.015 * TSP2, ng/ml) + (0.0016 * CD163, ng/ml)

**TSP2/IGFBP7/CD163**

- 3.08 + (0.014*TSP2, ng/ml) + (0.0005 * IGFBP7, ng/ml) + (0.0016 * CD163, ng/ml)

**Fig.21**     Patients with primary sclerosing cholangitis (PSC) with mild/moderate vs advanced liver fibrosis

F0-2 vs 3-4 (PSC)

Legend:
- TSP2 (0.825)
- IGFBP7 (0.768)
- CD163 (0.771)
- TSP4 (0.645)
- TSP2|IGFBP7|CD163 (0.833)
- TSP2|IGFBP7|CD163|TSP4 (0.835)

N= 115
F1 = 68   F2 = 9
F3 = 20   F4 = 18

**TSP2/IGFBP7/CD163**

- 3.94 + (0.018 * TSP2, ng/ml)+(0.009 * IGFBP7, ng/ml)+(0.0016 * CD163, ng/ml)

**TSP2/IGFBP7/CD163/TSP4**

- 3.94 + (0.018 * TSP2, ng/ml) + (0.0008 * IGFBP7, ng/ml) + (0.0016 * CD163, ng/ml) + (0.00004 * TSP2, ng/ml)

## Fig.22   Patients with alcohol-induced liver cirrhosis vs healthy controls

*N=63 (cirrhosis), 32 (HC)*

**Fig.23** Patients with compensated alcohol-induced cirrhosis vs healthy controls

Healthy Controls vs Child-Pugh A cirrhosis

*Healthy controls: N=30*
*Child-Pugh A: N=20*

**TSP2/IGFBP7**
-18.73 + (0.056 * TSP2, ng/ml) +
(0.06 * IGFBP7, ng/ml)
**TSP2/IGFBP7/CD163**
-1030.6+(5.01 * TSP2, ng/ml) +
(4.59 * IGFBP7, ng/ml) + (-0.53 *
CD163, ng/ml)

Europäisches
Patentamt

European
Patent Office

Office européen
des brevets

# EUROPEAN SEARCH REPORT

**Application Number**

EP 23 20 8165

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAMBABU SURABATTULA: "Novel matricellular serum markers predict liver fibrosis and fibrogenesis in patients with NAFLD undergoing bariatric surgery. Abstract no.2085-A", HEPATOLOGY, vol. 78, no. S1, 12 October 2023 (2023-10-12), page 854, XP093156613, DOI: 10.1097/HEP.0000000000000580 Retrieved from the Internet: URL:https://journals.lww.com/hep/fulltext/2023/10001/the_liver_meeting__boston,_massachusetts_nov.1.aspx#> * abstract * | 1-14 | INV. C12Q1/6883 G01N33/6893 G01N33/50 |
| X | RAMBABU SURABATTULA: "Serum thrombospondin 2 and insulin-like growth factor predict advanced liver fibrosis with common variable immunodeficiency", HEPATOLOGY, vol. 78, no. S1, 12 October 2023 (2023-10-12), page 1457, XP093156617, DOI: 10.1097/HEP.0000000000000580 Retrieved from the Internet: URL:https://journals.lww.com/hep/fulltext/2023/10001/the_liver_meeting__boston,_massachusetts_nov.1.aspx#> * abstract * | 1-7, 10-15 | |
| | ----- -/-- | | TECHNICAL FIELDS SEARCHED (IPC) C12Q G01N |

The present search report has been drawn up for all claims

| Place of search | Date of completion of the search | Examiner |
|---|---|---|
| Munich | 30 April 2024 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

# EUROPEAN SEARCH REPORT

Application Number

EP 23 20 8165

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAMBABU SURABATTULA: "Novel serum fibrosis markers to predict rapid liver fibrosis progression post liver transplant", HEPATOLOGY, vol. 78, no. S1, 12 October 2023 (2023-10-12), pages S1553-S1554, XP093156622, DOI: 10.1097/HEP.0000000000000580 Retrieved from the Internet: URL:https://journals.lww.com/hep/fulltext/2023/10001/the_liver_meeting__boston,_massachusetts_nov.1.aspx#> * abstract * | 1-6, 10-15 | |
| X | US 2020/340060 A1 (BILLIN ANDREW NICHOLAS [US] ET AL) 29 October 2020 (2020-10-29) * the whole document * | 1-6, 10-15 | |
| X | US 2023/194536 A1 (LIBERMANN TOWIA ARON [US] ET AL) 22 June 2023 (2023-06-22) * the whole document * | 1-7, 10-15 | **TECHNICAL FIELDS SEARCHED (IPC)** |
| A | EDUARDO VILAR-GOMEZ: "Non-invasive assessment of non-alcoholic fatty liver disease: Clinical prediction rules and blood-based biomarkers", JOURNAL OF HEPATOLOGY, vol. 68, no. 2, 1 February 2018 (2018-02-01), pages 305-315, XP093154119, AMSTERDAM, NL ISSN: 0168-8278, DOI: 10.1016/j.jhep.2017.11.013 * the whole document * | 1-15 | |

-/--

The present search report has been drawn up for all claims

| Place of search | Date of completion of the search | Examiner |
|---|---|---|
| Munich | 30 April 2024 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 23 20 8165

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2022/254239 A1 (UNIV OF HONG KONG [CN]) 8 December 2022 (2022-12-08) * the whole document * | 1-15 | |
| A | WO 2021/226042 A1 (UNIV FLORIDA [US]) 11 November 2021 (2021-11-11) * the whole document * | 1-15 | |
| A | TIMUR LIWINSKI: "A prospective pilot study of a gluten-free diet for primary sclerosing cholangitis and associated colitis", ALIMENTARY PHARMACOLOGY & THERAPEUTICS, vol. 57, no. 2, 20 October 2022 (2022-10-20), pages 224-236, XP093156852, GB ISSN: 0269-2813, DOI: 10.1111/apt.17256 Retrieved from the Internet: URL:https://onlinelibrary.wiley.com/doi/full-xml/10.1111/apt.17256> * abstract * | 1-15 | |

TECHNICAL FIELDS
SEARCHED        (IPC)

The present search report has been drawn up for all claims

| Place of search | Date of completion of the search | Examiner |
|---|---|---|
| Munich | 30 April 2024 | Bradbrook, Derek |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 3 of 3

## EP 4 553 173 A1

### ANNEX TO THE EUROPEAN SEARCH REPORT
### ON EUROPEAN PATENT APPLICATION NO.

EP 23 20 8165

30-04-2024

| Patent document cited in search report | | | Publication date | Patent family member(s) | | | Publication date |
|---|---|---|---|---|---|---|---|
| US 2020340060 | A1 | | 29-10-2020 | EP | 3710599 | A1 | 23-09-2020 |
| | | | | US | 2020340060 | A1 | 29-10-2020 |
| | | | | WO | 2019094777 | A1 | 16-05-2019 |
| US 2023194536 | A1 | | 22-06-2023 | CA | 3082591 | A1 | 23-05-2019 |
| | | | | EP | 3710831 | A1 | 23-09-2020 |
| | | | | US | 2023194536 | A1 | 22-06-2023 |
| | | | | WO | 2019099706 | A1 | 23-05-2019 |
| WO 2022254239 | A1 | | 08-12-2022 | CN | 117642630 | A | 01-03-2024 |
| | | | | EP | 4348261 | A1 | 10-04-2024 |
| | | | | WO | 2022254239 | A1 | 08-12-2022 |
| WO 2021226042 | A1 | | 11-11-2021 | US | 2023203583 | A1 | 29-06-2023 |
| | | | | WO | 2021226042 | A1 | 11-11-2021 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017139254 A1
- WO 2019099706 A1
- WO 2022014580 A1

**Non-patent literature cited in the description**

- **KARSDAL MA ; KRAUS VB ; SHEVELL D ; BAY-JENSEN AC ; SCHATTENBERG J ; SURABATTULA R ; SCHUPPAN D.** Profiling and targeting connective tissue remodeling in autoimmunity - A novel paradigm for diagnosing and treating chronic diseases. *Autoimmun Rev*, 2021, vol. 20, 102706
- **SCHUPPAN D ; MYNENI S ; SURABATTULA R.** Liquid biomarkers for fibrotic NASH - progress in a complex field.. *J Hepatol*, 2022, 76-7
- **WATTACHERIL JJ ; ABDELMALEK MF ; LIM JK ; SANYAL AJ.** AGA Clinical Practice Update on the Role of Noninvasive Biomarkers in the Evaluation and Management of Nonalcoholic Fatty Liver Disease: Expert Review. *Gastroenterology*, 2023, vol. 165, 1080-88
- **SANYAL AJ ; CASTERA L ; WONG VW.** Non-invasive Assessment of Liver Fibrosis in NAFLD. *Clin Gastroenterol Hepatol*, 2023, vol. 21, 2026-39